# EUROPEAN PATENT APPLICATION

(11) **EP 4 574 146 A1**
(43) Date of publication of application: **25.06.2025**
(21) Application number: 23383314.4
(22) Date of filing: 18.12.2023
(51) Int. Cl.: A61K 31/198, A61K 9/00, A61P 25/00, A61P 27/06

(54) **DHP-I INHIBITORS FOR USE AS NEUROPROTECTANTS AND IN THE TREATMENT OF NEUROINFLAMMATORY DISEASES**

(71) Applicant: Telara Pharma S.L., 28001 Madrid (ES); Fundación para la Investigación Biomédica del Hospital Gregorio Marañón (FIBHGM), 28007 Madrid (ES); Universidad Complutense De Madrid, 28040 Madrid (ES)
(72) Inventor: LÁZARO FERNÁNDEZ, Alberto, 28040 Madrid (ES); LÓPEZ GALLARDO, Meritxell, 28040 Madrid (ES); GONZÁLEZ-NICOLÁS GONZÁLEZ, María Ángeles, 28007 Madrid (ES); MARCO LÓPEZ, Eva María, 28040 Madrid (ES); MARTÍN SÁNCHEZ, Beatriz, 28007 Madrid (ES); SAN FELIPE RIBA, Diego, 28040 Madrid (ES); LLORENTE DE MIGUEL, Ricardo, 28040 Madrid (ES); MARTÍNEZ LÓPEZ, Miguel Ángel, 28040 Madrid (ES); RUBIO CASADO, Sara, 28040 Madrid (ES)
(74) Representative: Hoffmann Eitle

(57) **Abstract**

The present invention relates to a DHP-I inhibitor for use in a method for the prophylactic and/or therapeutic treatment of a neuroinflammatory disease. In some embodiments, it relates to a DHP-I inhibitor for use in a method for the prophylactic treatment of neurodegeneration. The present invention also relates to a DHP-I inhibitor for use as neuroprotectant. It further pertains to related methods for the prophylactic and/or therapeutic treatment of a neuroinflammatory disease and the use of a DHP-I inhibitor in the manufacturing of a medicament for use in these methods. The present invention also provides pharmaceutical compositions and delivery systems for use in the methods of the invention, as well as to a pharmaceutical composition or drug delivery systems suitable for administration by a route bypassing the blood brain barrier (BBB) and/or the blood retinal barrier (BRB), preferably selected intrathecal, intracranial, intranasal and/or ocular administration; or suitable for trespassing the BBB and/or the BRB.

## Description

### FIELD OF INVENTION

The present invention relates to the medical and pharmaceutical field, in particular to the research and development of new drugs for the treatment of neuroinflammatory diseases, wherein a neuroinflammatory disease is a neurological or mental disease of the central nervous system (CNS) and/or the peripheral nervous system (PNS), which pathogenesis involves primary or secondary neuroinflammation and may evolve into neurodegeneration. Examples of neuroinflammatory diseases characterized by neurodegeneration are for instance Alzheimer's and Parkinson's diseases, amyotrophic lateral sclerosis, Huntington's disease, spinocerebellar ataxia, multiple sclerosis and ocular neurodegenerative diseases, such as glaucoma.

More specifically, the present invention relates to a DHP-I inhibitor for use in a method for the prophylactic and/or therapeutic treatment of a neuroinflammatory disease. In some embodiments, it relates to a DHP-I inhibitor for use in a method for the prophylactic treatment of neurodegeneration. The present invention also relates to a DHP-I inhibitor for use as neuroprotectant. It further pertains to related methods for the prophylactic and/or therapeutic treatment of a neuroinflammatory disease and the use of a DHP-I inhibitor in the manufacturing of a medicament for use in these methods. The present invention also provides pharmaceutical compositions and delivery systems for use in the methods of the invention, as well as to a pharmaceutical composition or drug delivery systems suitable for administration by a route bypassing the blood brain barrier (BBB) and/or the blood retinal barrier (BRB), preferably selected from intrathecal, intracranial, intranasal and/or ocular administration; or a pharmaceutical composition or drug delivery systems suitable for trespassing the BBB and/or the BRB.

### BACKGROUND OF THE INVENTION

A neuroinflammatory state is a shared characteristic of acute and chronic neurological diseases (Lyman et al., 2014). At first, neuroinflammation can act as a neuroprotective mechanism, but sustained neuroinflammation can induce neurotoxicity and neurodegeneration.

Neuroinflammation occurs in the PNS (that is, peripheral nerves and ganglia) and CNS (that is, the spinal cord and brain) and is characterized by infiltration of leukocytes and increased production of inflammatory mediators at these sites (Ji RR et al. 2014; Stephenson J. et al. 2018, Marinelli S. et al. 2021). Microglia and macroglia cells are the main cells that play a role in said inflammatory process. Pro-inflammatory mediators can go through the blood brain barrier and recruit leucocytes into the brain (Persidsky *et al.,* 2006). At first, the inflammatory response may promote tissue repair and remove cellular debris. However, a sustained response may have negative effects and inhibit tissue regeneration. Neuroinflammatory processes alter the homeostatic environment and the synaptic function, leading to neuronal cell death and worsen several neurodegenerative diseases (Cunningham *et al.,* 1996). Both endogenous (e.g., genetic mutations and protein aggregation) and environmental factors (e.g., infections, trauma and drugs) may be the cause of these continuous inflammatory processes (Glass *et al.,* 2010). Neurodegenerative diseases are a growing worldwide health problem that leads to patients physical and mental disabilities, or even death. There is an increasing field of studies focused on slowing the progression of these neurodegenerative diseases, as well as attenuating the symptoms they cause.

The retina is the neuro-sensory epithelium that belongs to the CNS and is located between the eye's vitreous humour and the choroid. It is responsible for the process of phototransduction, where light, which is formed by electromagnetic waves, becomes electric energy in the form of nervous impulses. Glaucoma is a multifactorial neurodegenerative disease of the retina, characterized by the irreversible death of a type of neuron located near the inner surface of the retina towards the vitreous humour called retinal ganglion cells (RGCs), which leads to the progressive degeneration of their axons, causing irreversible blindness. Notably, since glaucoma emerges gradually, it frequently appears as asymptomatic, and, whenever the vision loss becomes evident, the retinal damage is already irreversible (Mélik Parsadaniantz *et al.,* 2020). Glaucoma is one of the first causes of irreversible blindness in the world: It affects around 60 million of people, 8.4 million of which are already blind. These data are alarmingly growing, and the number of glaucomatous patients is expected to reach 111.8 million by 2040 (Lago & Bengoa, 2018, Flaxman, S. R., 2017).

Glaucoma is a multifactorial pathology, highly influenced by both genetic and environmental factors. Among risk factors, an abnormal intraocular pressure (IOP) is stated as the most frequent cause, followed by vascular deregulation, neuroinflammation, oxidative stress, excitotoxicity, hypoxia, age, family history and race. IOP depends on the aqueous humour, which is constantly produced by the ciliary bodies, released into the eye's posterior chamber, then flows towards the anterior chamber, and is drained, through the trabecular meshwork and the Schlemm's channel, into the episcleral and limbar veins. An increase in IOP can result from both an increase of aqueous humour production and/or a deficiency in its drainage. If aqueous humour drainage is blocked by an obstruction of the trabecular pathway, in case of a normal iridocorneal angle, a direct increase in IOP is reported, and a primary open-angle glaucoma is diagnosed. The primary open-angle glaucoma is nowadays the most common glaucoma (Jonas *et al.,* 2017;). Abnormal IOP may exert mechanical pressure over the optic disc, inducing the direct deformation of RGCs axons, atrophy in the optic nerve and possibly RGCs death due to anterograde and retrograde signalling (Jonas *et al.,* 2017).

At present, there is no effective treatment for glaucoma, and the gold standard in its management involves lowering the IOP. IOP-lowering treatments, however, aim to reduce 20-50% of the IOP, and even if the lOP is effectively controlled, the vast majority of patients keep experiencing vision loss (Cordeiro and Levin, 2011). What is more, there is a subgroup of primary open angle glaucoma, called normotension or normal tension glaucoma (NTG) that comprises up to 30% of glaucoma in the western hemisphere and up to 90% in the far east, in which IOP is normal or even within the lower normal range and develop vision loss (Cho and Kee, 2014; Klein *et al.,* 1992). Therefore, glaucoma, in addition to an elevated IOP, may be due to other causes, among which a proinflammatory environment has been described: controlling it may help in the management of glaucoma and its progression.

Cilastatin was first described as a competitive inhibitor of renal dehydropeptidase I (DHP-I) which prevented hydrolysis of the peptide bond and opening of lactam rings. In the presence of cilastatin, DHP-I did not open the lactam ring of imipenem, prevented its absorption, and increased urinary excretion of imipenem, reducing its concentration in tubular cells (Clissold *et al*., 1987). Thus, cilastatin has traditionally been used in combination with imipenem (a betalactam antibiotic) in order to protect it from DHP-I and prolong its antibacterial effect. However, cilastatin itself does not have any antibacterial activity. For instance, it has been commercialized in Spain under the trade name Tienam^{®} as a formulation for intravenous administration containing imipenem / cilastatin (1:1) in doses of 500 mg. Tienam^{®} has been approved for the treatment of infections in adults and children of 1 year of age and above including complicated intra-abdominal infections; severe pneumonia including hospital and ventilator-associated pneumonia; intra- and post-partum infections; complicated urinary tract infections; and complicated skin and soft tissue infections. It has been further approved for the treatment of patients with bacterial septicemia that occurs in association with or is suspected to be associated with any of said infections. More recently, cilastatin has been approved in combination with imipenem and relebactam under the trade name Recarbrio^{®} (MSD). It is indicated in Europe for the treatment of hospital-acquired pneumonia (HAP), treatment of bacteraemia in association with HAP and infections caused by Gram-negative organisms (Recarbrio^{®} EPAR, EMA). The FDA approved Recarbrio^{®} for complicated urinary tract infections, including pyelonephritis and complicated intra-abdominal infections in 2019 (Recarbrio^{®} Label, FDA).

Cilastatin has also been described as capable of reducing renal damage of nephrotoxic drugs other than beta-lactams (WO 97/37649). More specifically, cilastatin was reported to have beneficial effects *in vitro* and *in vivo* against common nephrotoxic agents used in human clinical settings such as cisplatin, cyclosporine A, vancomycin or gentamicin without reducing their therapeutic activity (Humanes B *et al.,* 2012, Jado JC et al, 2020). Indeed, in a previous work, the inventors reported that cilastatin is an inhibitor of DHP-I which is present in the cholesterol lipid rafts of the apical brush border of renal proximal tubular epithelial cells (PTECs) (Camano S et al., 2010), which lead to the reduction or cancellation of key steps of the extrinsic pathway of apoptosis, with a protective effect against oxidative stress and cell death by apoptosis induced by nephrotoxic agents (Humanes *B et al.,* 2012, Jado JC et al, 2020). In a subsequent work, the inventors described that cilastatin was successful in preventing and treating sepsis and sepsis-induced renal injury (WO2017220810A1).

Currently, there are no therapeutic alternatives that have demonstrated great efficacy in the treatment of neuroinflammatory diseases. Therefore, there is an urgent need to find new safe and effective drugs for their prevention and/or treatment.

### SUMMARY OF THE INVENTION

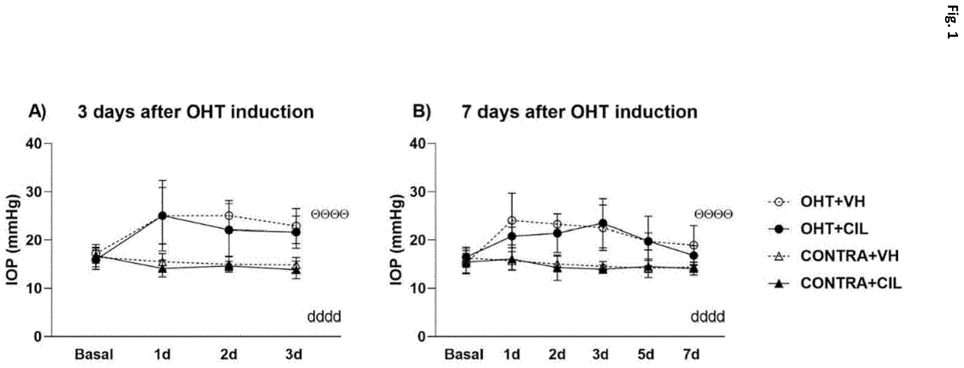

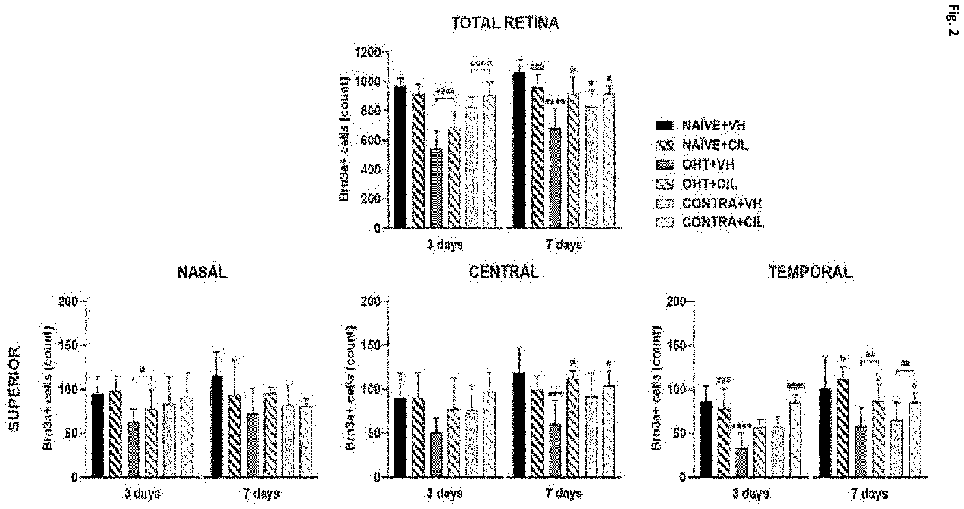

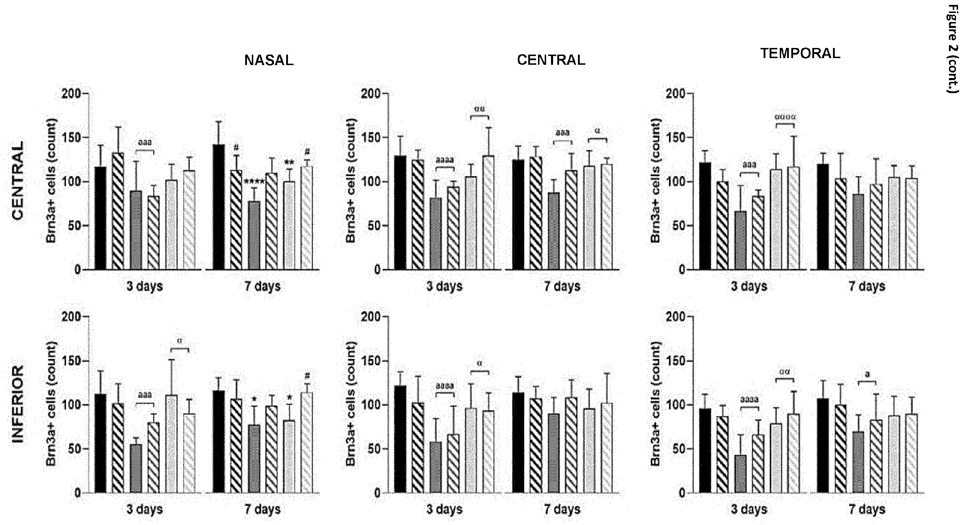

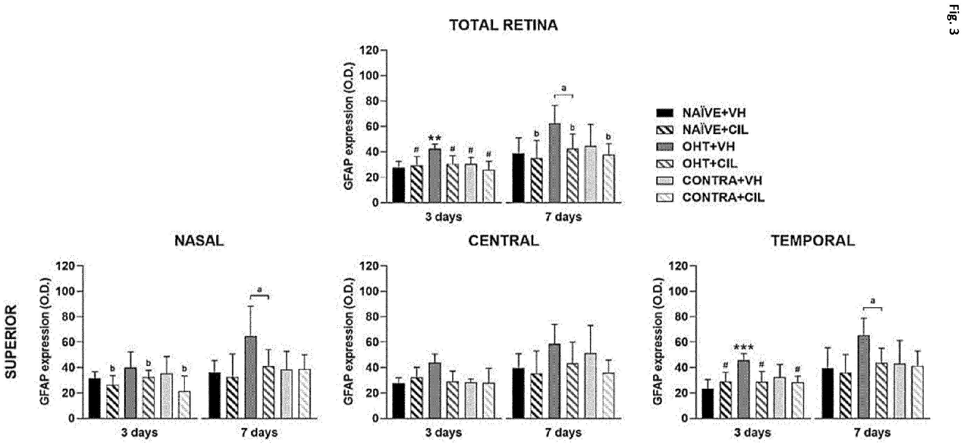

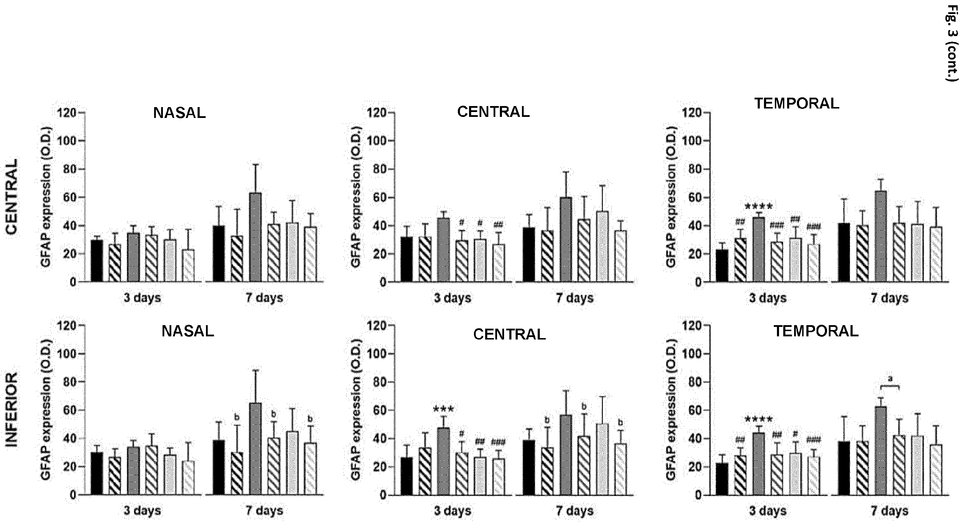

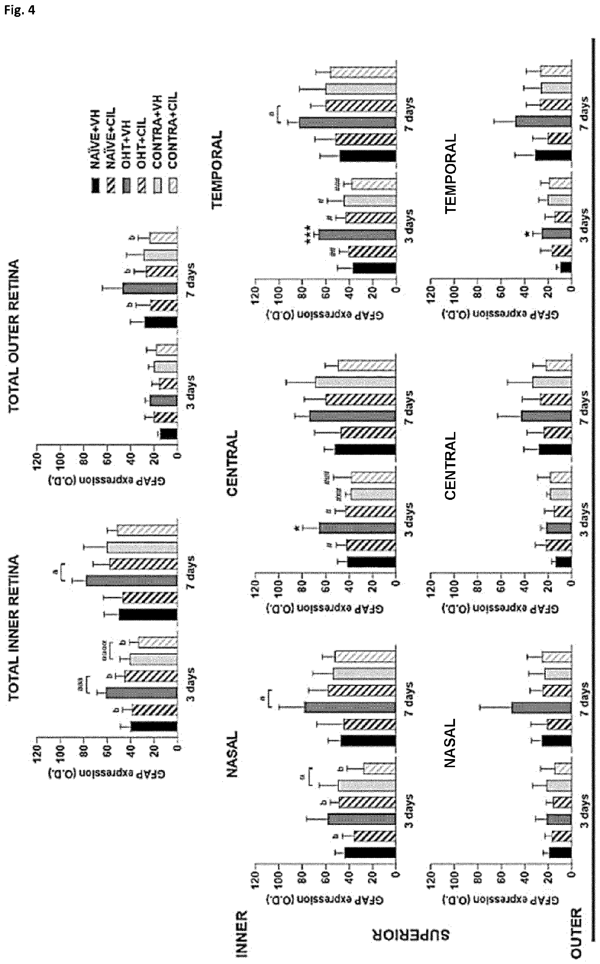

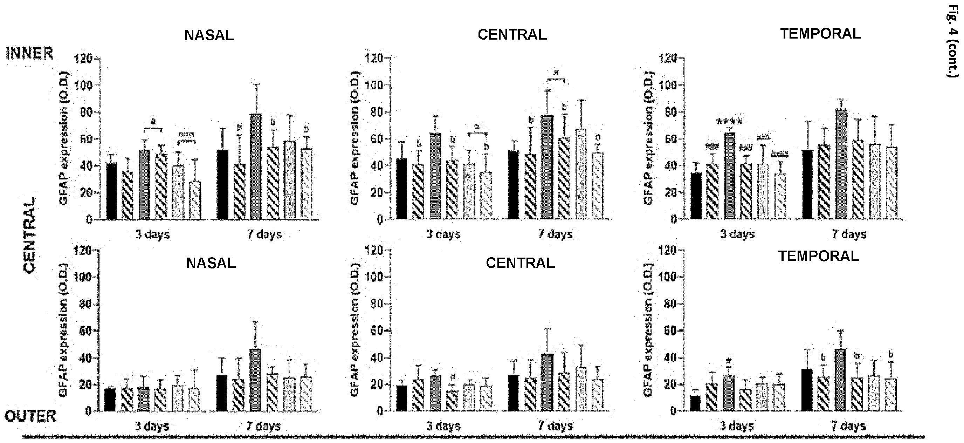

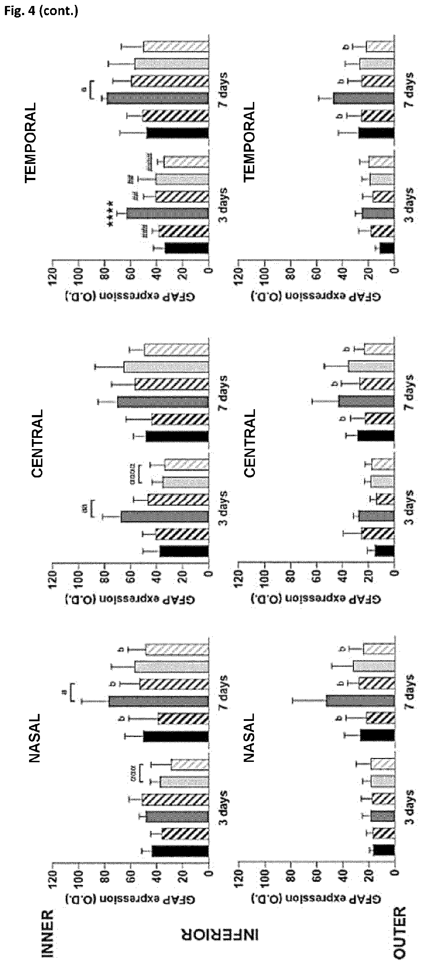

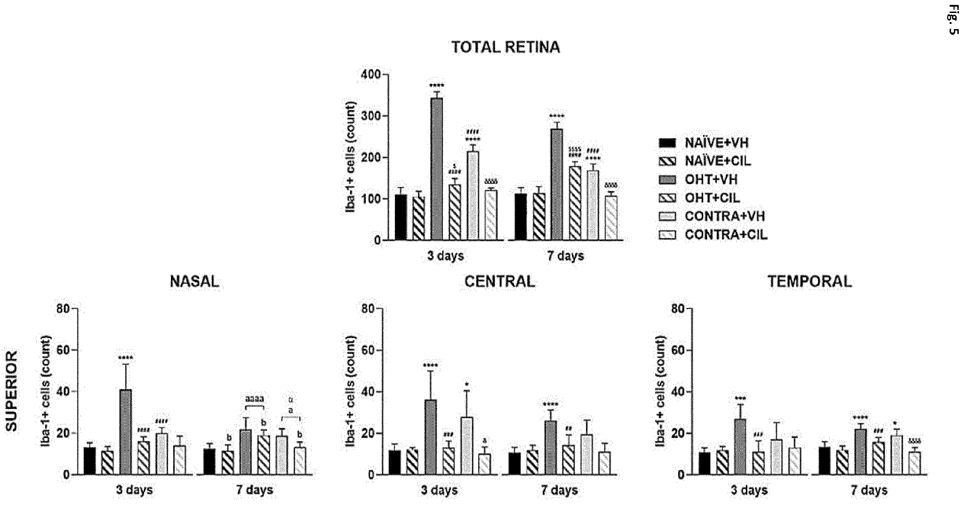

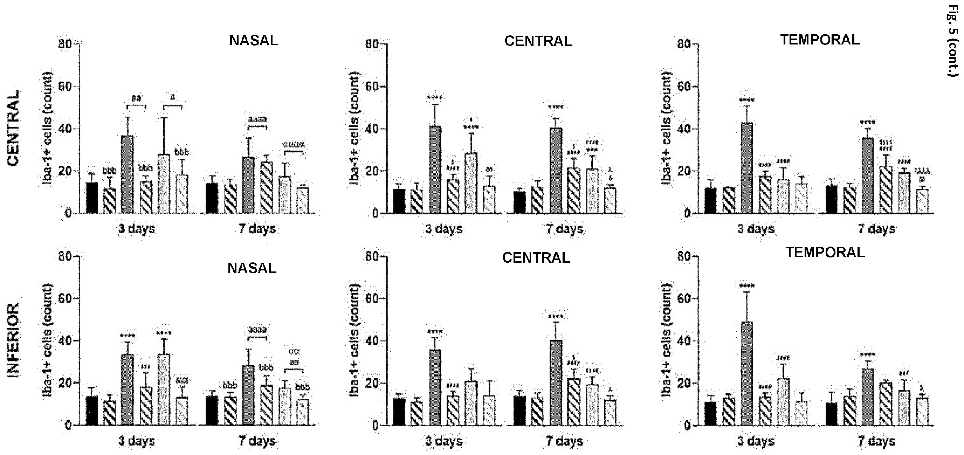

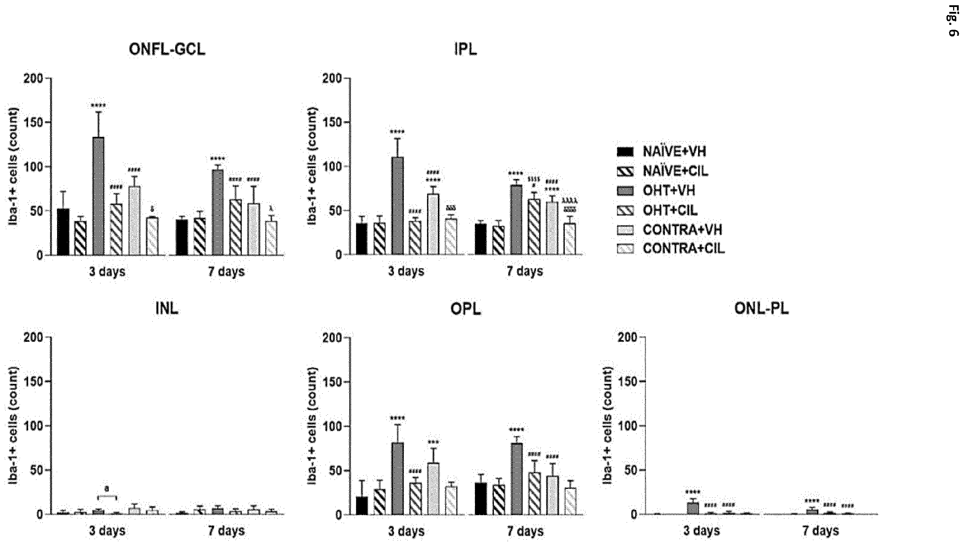

The inventors have found that the DHP-I inhibitor cilastatin surprisingly exerted a protective role in neuroinflammatory conditions. As a proof-of-concept the inventors used a glaucoma model based on unilateral laser induced ocular hypertension (OHT). Glaucoma is a group of diseases characterized by progressive optic nerve degeneration and is recognized as a neurodegenerative disease associated with neuroinflammation (Marchesi et al. 2021). It was found by the research group that the tested DHP-I inhibitor protected the retina, without modifying the lOP value, demonstrating that the mechanism of action of cilastatin did not involve a decrease of lOP (Fig. 1). Unexpectedly, the present inventors found that the administration of a DHP-I inhibitor (i.e., cilastatin) was capable of partially avoiding the loss of retinal ganglion cells (RGCs) and to reduce the inflammatory response mainly of microglial but also of macroglial cells in the glaucoma model, providing the following beneficial effects:
(a) attenuating the reduction of RGCs (Brn3a+ cells), as shown in Fig. 2 the Brn3a+ cell numbers in the CIL treated group was maintained close to the NAÏVE eyes group;
(b) decreasing the inflammatory response of the macroglia, as shown by a reduction of the expression of Glial fibrillary acidic protein (GFAP) in macroglial cells (Figs. 3 and 4); and
(c) decreasing the inflammatory response of the microglia, as shown by a reduction of Iba-1 levels (Figs. 5 and 6).

Interestingly, a reduction of the inflammatory response of the microglia (Figs.3 and 4) and macroglia (Figs.5 and 6) was not only observed in the OHT eye, where inflammation was secondary to the laser-induced ocular hypertension, but also in the CONTRA eye where there was no IOP value changes (Fig. 1), but signs of inflammation were observed in the untreated mice (i.e., primary inflammation without mechanical damage).

The above results support that DHP-I inhibitors can be suitable for the treatment and/or prevention of neuroinflammatory diseases and neurodegeneration. Without willing to be bound by theory, the above results suggest that the treatment by cilastatin of the neuroinflammatory state or pathology characterizing the neuroinflammatory disease is preventing neurodegeneration.

Altogether, the neuroprotective results shown in glaucoma, where cilastatin was found to decrease the progressive loss of RCGs, and the decrease of the glia-mediated neuroinflammatory response, supports the generalization of the observed effects to DHP-I inhibitors and to other neuroinflammatory diseases.

Accordingly, the first aspect of the invention relates to a DHP-I inhibitor for use as a neuroprotective medicament.

In a second aspect, the present invention provides a DHP-I inhibitor for use in a method for the prophylactic and/or therapeutic treatment of a neuroinflammatory disease in a mammalian subject.

In a related aspect, it provides a method for the prophylactic and/or therapeutic treatment of a neuroinflammatory disease in a mammalian subject, comprising the administration of therapeutically effective amount of a DHP-I inhibitor to a subject in need thereof. Moreover, it pertains to the use of a DHP-I inhibitor in the manufacture of a medicament for the prophylactic and/or therapeutic treatment of a neuroinflammatory disease.

In a third aspect, the invention pertains to a composition comprising a DHP-I inhibitor and a pharmaceutically acceptable excipient, for use according to the first or the second aspects. In preferred embodiments, the pharmaceutical composition is according to the fifth or sixth aspects.

In a fourth aspect, the invention pertains to a drug delivery system comprising a DHP-I inhibitor or the pharmaceutical composition comprising thereof as described herein, for use according to the first or the second aspects. In preferred embodiments, the drug delivery system is according to the fifth or sixth aspects.

In a fifth aspect, the invention provides a pharmaceutical composition comprising a DHP-I inhibitor and a pharmaceutically acceptable excipient, or a drug delivery system comprising a DHP-I inhibitor or the pharmaceutical composition comprising thereof as described herein, wherein the pharmaceutical composition or drug delivery system is suitable for trespassing the blood brain barrier (BBB) and/or the blood retinal barrier (BRB).

In a sixth aspect, the invention relates to a pharmaceutical composition comprising a DHP-I inhibitor and a pharmaceutically acceptable excipient, or a drug delivery system comprising a DHP-I inhibitor or the pharmaceutical composition comprising thereof, wherein the pharmaceutical composition or drug delivery system is suitable for administration by a route bypassing the BBB and/or BRB, preferably selected from intrathecal, intracranial, intranasal and/or ocular administration, including topical, intraocular and/or periocular administration.

### BRIEF DESCRIPTION OF THE DRAWINGS

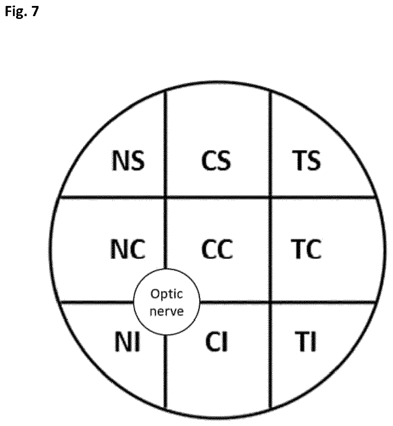

**Figure 1****. Evolution of Intraocular Pressure (IOP) measurements.** IOP was measured in the left photocoagulated eye (laser-induced ocular hypertension, OHT), and its contralateral (CONTRA). Animals were administered with CIL (300 mg/kg, i.p.) or vehicle (VH, saline) from two days before the surgery and until sacrifice A) 3 or B) 7 days after surgery, i.e., animals received 6 or 10 injections. Experimental groups: OHT+VH day 3 (n=12), OHT+CIL day 3 (n=11), CONTRA+VH day 3 (n=12), CONTRA+CIL day 3 (n=11); OHT+VH day 7 (n=11), OHT+CIL day 7 (n=9), CONTRA+VH day 7 (n=11), CONTRA+CIL day 7 (n=9) Repeated Measures ANOVA: dddd p<0.001, general effect of the day; ΘΘΘΘ p<0.001, general effect of the surgical intervention.
**Figure 2****. Evolution of brain-specific homeobox/POU domain protein 3A (Brn3a) + cells count in the retinal areas.** Analysis of the total Brn3a+ cells in the 9 retinal areas, 3 and 7 days after the OHT-induction in the different experimental groups. Experimental groups (n=5): NAÏVE+VH day 3, NAÏVE+CIL day 3, OHT+VH day 3, OHT+CIL day 3, CONTRA+VH day 3, CONTRA+CIL day 3; NAÏVE+VH day 7, NAÏVE+CIL day 7, OHT+VH day 7, OHT+CIL day 7, CONTRA+VH day 7, CONTRA+CIL day 7. Two-way ANOVA, in case of a general effect of the surgery: a p<0.05, aa p<0.01, aaa p<0.005, aaaa p<0.001 vs NAÏVE group; α p<0.05, αα p<0.01, ααα p<0.005, αααα p<0.001 vs OHT group; in case of a significant interaction between factors, post-hoc comparisons were performed using the Tukey test: *p<0.05, **p<0.01, ***p<0.005, ****p<0.001 vs NAÏVE+VH; # p<0.05, ## p<0.01, ### p<0.005, #### p<0.001 vs OHT+VH; b p<0.05 general effect of the pharmacological treatment.
**Figure 3****. Evolution of glial fibrillary acidic protein (GFAP) + cells count in the retinal areas.** GFAP+ cells in the 9 retinal areas, 3 and 7 days after the OHT-induction in the different experimental groups. Experimental groups (n=5): NAÏVE+VH day 3, NAÏVE+CIL day 3, OHT+VH day 3, OHT+CIL day 3, CONTRA+VH day 3, CONTRA+CIL day 3; NAÏVE+VH day 7, NAÏVE+CIL day 7, OHT+VH day 7, OHT+CIL day 7, CONTRA+VH day 7, CONTRA+CIL day 7. Two-way ANOVA, in case of a general effect of the surgery: a p<0.05, vs NAÏVE group; general effect of the pharmacological treatment: b p<0.05; in case of a significant interaction between factors, post-hoc comparisons were performed using the Tukey test: **p<0.01, ***p<0.005, ****p<0.001 vs NAÏVE+VH; # p<0.05, ## p<0.01, ### p<0.005, vs OHT+VH.
**Figure 4****. Evolution of glial fibrillary acidic protein (GFAP) + cells count in the retinal areas of the inner and outer retina.** GFAP+ cells in the 9 inner (blue) and outer (green) retinal areas, 3 and 7 days after the OHT-induction in the different experimental groups. Experimental groups (n=5): NAÏVE+VH day 3, NAÏVE+CIL day 3, OHT+VH day 3, OHT+CIL day 3, CONTRA+VH day 3, CONTRA+CIL day 3; NAÏVE+VH day 7, NAÏVE+CIL day 7, OHT+VH day 7, OHT+CIL day 7, CONTRA+VH day 7, CONTRA+CIL day 7. Two-way ANOVA, in case of a general effect of the surgery: a p<0.05, aa p<0.01, aaa p<0.005 vs NAÏVE group; α p<0.05, aaa p<0.005, αααα p<0.001 vs OHT group; general effect of the pharmacological treatment: b p<0.05; in case of a significant interaction between factors, post-hoc comparisons were performed using the Tukey test: *p<0.05, ***p<0.005, ****p<0.001 vs NAÏVE+VH; # p<0.05, ## p<0.01, ### p<0.005, #### p<0.001 vs OHT+VH.
**Figure 5****. Evolution of ionized calcium-binding adapter molecule 1 (lba-1) + cells count in the retinal areas.** Iba-1+ cells in the 9 retinal areas, 3 and 7 days after the OHT-induction in the different experimental groups. Experimental groups (n=5): NAÏVE+VH day 3, NAÏVE+CIL day 3, OHT+VH day 3, OHT+CIL day 3, CONTRA+VH day 3, CONTRA+CIL day 3; NAÏVE+VH day 7, NAÏVE+CIL day 7, OHT+VH day 7, OHT+CIL day 7, CONTRA+VH day 7, CONTRA+CIL day 7. Two-way ANOVA, in case of a general effect of the surgery: a p<0.05, aa p<0.01, aaaa p<0.001 vs NAÏVE group; α p<0.05, αα p<0.01, αααα p<0.001 vs OHT group; general effect of the pharmacological treatment: b p<0.05, bbb p<0.005 ; in case of a significant interaction between factors, post-hoc comparisons were performed using the Tukey test: *p<0.05, ***p<0.005, ****p<0.001 vs NAÏVE+VH; # p<0.05, ## p<0.01, ### p<0.005, #### p<0.001 vs OHT+VH; $ p<0.05, $$$$ p<0.001 vs NAÏVE+CIL; δ p<0.05, δδ p<0.01, δδδδ p<0.001 vs CONTRA+VH; A p<0.05, *λλλλ* p<0.001 vs OHT+CIL.
**Figure 6****. Evolution of ionized calcium-binding adapter molecule 1 (lba-1) + cells count in the retinal layers.** lba-1+ cells in the optic nerve fibers layer-ganglion cells layer (ONFL-GCL, measured together as a unique layer), inner plexiform layer (IPL), inner nuclear layer (INL), outer plexiform layer (OPL), outer nuclear layer-photoreceptor layer (ONL-PL, measured together as an unique layer), 3 and 7 days after the OHT-induction in the different experimental groups. Experimental groups (n=5): NAÏVE+VH day 3, NAÏVE+CIL day 3, OHT+VH day 3, OHT+CIL day 3, CONTRA+VH day 3, CONTRA+CIL day 3; NAÏVE+VH day 7, NAÏVE+CIL day 7, OHT+VH day 7, OHT+CIL day 7, CONTRA+VH day 7, CONTRA+CIL day 7. Two-way ANOVA, in case of a general effect of the surgery: a p<0.05 vs NAÏVE group; in case of a significant interaction between factors, post-hoc comparisons were performed using the Tukey test: ***p<0.005, ****p<0.001 vs NAÏVE+VH; #### p<0.001 vs OHT+VH; $$$$ p<0.001 vs NAÏVE+CIL; δ p<0.05, δδ p<0.01, δδδ p<0.005, δδδδ p<0.001 vs CONTRA+VH; A p<0.05, *λλλλ* p<0.001 vs OHT+CIL.
**Figure 7****. Retinal areas:** Nasal (N), Central (C), Temporal (T), Superior (S), Inferior (I) and optic nerve.

### DETAILED DESCRIPTION OF THE INVENTION

### Definitions

Terms not specifically defined herein should be given the meanings that would be given to them by one of skill in the art in light of the disclosure and the context. As used in the specification, however, unless specified to the contrary, the following terms have the meaning indicated and the following conventions are adhered to.

Throughout the present specification and the accompanying clauses, the words "comprise" and variations such as "comprises", "comprising" are to be interpreted inclusively. That is, these words are intended to convey the possible inclusion of other elements or integers not specifically recited, where the context allows. The word "comprise" also includes the term "consists of".

The term "DHP-I inhibitor" refers to an agent that inhibits the dehydropeptidase-I (DHP-I) enzyme. DHP-I, also known as "dehydropeptidase I", "DPEP1", "membrane dipeptidase", "microsomal dipeptidase", or "renal dipeptidase", is a zinc-dependent metalloproteinase that hydrolyzes a variety of dipeptides and is involved in glutathione metabolism. It is a zinc-containing homodimer with a subunit M, of 47 000 to 59 000, that is anchored to the membrane with a glycosyl-phosphatidylinositol moiety. DHP-I preferentially catalyzes the hydrolysis of dipeptides with L-amino acids in the N-terminal position and both D- and L-peptides at the C-terminal position, e.g. Gly-L-Ala, Gly-D-Ala.

The inhibition of DHP-I means that the agent decreases DHP-I activity. DHP-I is inhibited by chelating agents, by transition-state analogues, and by substrate analogues. 1,10-Phenanthroline, penicillamine, and dithiothreitol inhibit porcine and human kidney DHP-I, either by chelating zinc or by binding to a zinc coordination site; activity is partially restored by incubation with ZnCl₂.

Preferably, the DHP-I inhibitor can selectively decrease DHP-I activity while having little or no effect on the activity of other enzymes. DHP-I activity can be determined following any of the protocols already known by those skilled in the art, such as the DHP-I activity assay described in Camano S. et al. 2010, where Gly-Phe-pnitroanilide was used as substrate for DHP-I activity determination. In one embodiment, the DHP-I inhibitor is a competitive inhibitor which prevents binding to DHP-I.

In one embodiment, the DHP-I inhibitor disclosed herein is an antagonist of the target, i.e., blocks or dampens a biological response by binding to and blocking the receptor so as to disrupt the interaction and inhibit the function of an agonist or inverse agonist. In a particular embodiment, the DHP-I inhibitor is a competitive antagonist, i.e., competes with an agonist for the active site. In another particular embodiment, the DHP-I inhibitor is a non-competitive antagonist, i.e., binds at a site other than the active site.

Illustrative non-limitative DHP-I inhibitors suitable in the context of the invention are cilastatin, bestatin, amastatin, peptides (such as those disclosed in WO2020109864, whose content is incorporated herein by reference; or JBP485 which is a cyclo-trans-4-L-hydroxyprolyl-L-serine dipeptide) and any pharmaceutically acceptable salt thereof. In some embodiments, the DHP-I inhibitor is characterized by reducing or preventing cell apoptosis, such as cilastatin or JBP485 (Wang et al. 2022)

In the context of the present invention, the term "salt" must be understood as any form of a compound used in accordance with this invention in which said compound is in ionic form or is charged and coupled to a counter-ion (a cation or anion) or is in solution. This definition also includes quaternary ammonium salts and complexes of the active molecule with other molecules and ions, particularly, complexes formed via ionic interactions. The definition includes in particular physiologically acceptable salts; this term must be understood as equivalent to "pharmacologically acceptable salts" or "pharmaceutically acceptable salts".

In the context of the present invention, the term "pharmaceutically acceptable salt" means any salt that is tolerated physiologically (normally meaning that it is not toxic, particularly, as a result of the counter-ion) when used in an appropriate manner for a treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion and at least one physiologically tolerated cation, preferably inorganic, particularly when used in humans and/or mammals. Salts with alkali and alkali earth metals are preferred particularly, as well as those formed with ammonium cations (NH₄⁺). Preferred salts are those formed with (mono) or (di)sodium, (mono) or (di)potassium, magnesium or calcium. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals. This definition specifically includes in the context of this invention a salt formed by a physiologically tolerated acid, i.e., salts of a specific active compound with physiologically tolerated organic or inorganic acids - particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

In one embodiment the DHP-I inhibitor is cilastatin or a pharmaceutically acceptable salt thereof. The term "cilastatin" as used herein refers to (Z)-7-[(2R)-2-Amino-3-hydroxy-3-oxopropyl]sulfanyl-2-{[(1 S)-2,2-dimethylcyclopropanecarbonyl]amino}hept-2-enoic acid of formula:

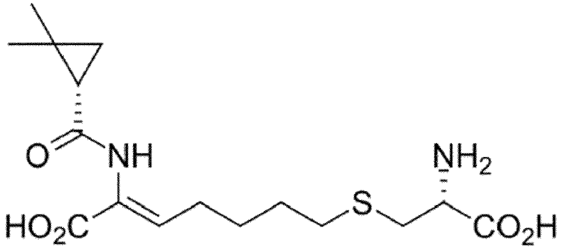

It encompasses the crystalline form and any pharmaceutical salts, solvates or prodrugs thereof. Typically, cilastatin has been used in the sodium salt form. Cilastatin and related dehydropeptidase-I (DHP-I) inhibitors are disclosed for example in US 4,668,504 which is herein incorporated by reference. In a particular embodiment, the DHP-I inhibitor is sodium cilastatin salt.

The term "neuroinflammatory disease" are defined as conditions involving an inflammatory response within the CNS and/or the PNS. Such inflammation (also referred herein as "neuroinflammation") can be caused by several pathological stimuli, which can comprise infections, trauma, toxins and ischemia. Proinflammatory cytokines are produced, comprising IL-1β, IL-18, IL-6 and tumor necrosis factor (TNF), as well as chemokines comprising C-C motif chemokine ligand 1 (CCL1), CCL5 and CXC motif chemokine ligand 1 (CXCL1), small-molecule mediators like nitric oxide (NO) and prostaglandins and reactive oxygen species (ROS), being responsible for this production the cells of the innate immunity in the CNS and/or the PNS. The main players of the innate immunity are resident glia, microglia and astrocytes, although infiltrating blood cells and capillary endothelial cells can also contribute to this inflammatory process, when there is the damage on the BBB. The context, duration and course of the primary insult impacts on the degree of neuroinflammation. This process may involve the recruitment of immune cells, edema, tissue damage and cell death (DiSabato *et al.,* 2016). Increased vascular permeability, leukocyte infiltration, glial cell activation and increased cytokines and chemokines production are characteristics of neuroinflammation. A "neuroinflammatory disease" can be defined as a neurological disease characterized by a neuroinflammatory state or pathology. In preferred embodiments, it refers to condition or disorder where there is inflammation of the CNS. The terms "neuroinflammatory disease", "neuroinflammatory disorder", "neuroinflammatory condition" and "neuroinflammatory pathology" can be used herein interchangeably.

The term "neurodegeneration" refers to the progressive degeneration of the structure and/or function of the CNS and/or the PNS. Chronic microglial and astroglial activation and the production of pro-inflammatory cytokines are processes occurring in neuroinflammation that will often result in neurodegeneration.

The term "neuroprotection" describes the attenuation, reversion, delay or prevention of neurodegeneration. A "neuroprotectant" or "neuroprotective agent or medicament" attenuates, reverses, delays or prevents neurodegeneration.

"Glaucoma" is defined as a group of optic neuropathies featured by the progressive degeneration of retinal ganglion cells, which leads to changes in the optic nerve, and vision loss due to retinal ganglion cells axon degeneration. It is a multifactorial disease process which may involve high intraocular pressure (IOP), abnormal ocular blood flow, abnormal structural susceptibility of the lamina cribrosa, low intracranial pressure, autoimmunity and/or mitochondrial function impairment (Weinreb *et al*., 2014, Kang and Tana, 2021).

"Neurons" or "nerve cells" are excitable cells that can transduce a wide variety of stimuli into electrical signals and transmit these signals to other nervous cells. They continuously send information about the external and internal environment to the central nervous system (Fletcher, 2011) and within the central nervous system, they integrate and process all the information received, generating responses if necessary. The nervous system has been divided into the CNS, comprised by the brain and the spinal cord, and the PNS. The PNS is integrated by the autonomic nervous system (sympathetic and parasympathetic), the enteric nervous system and the somatic nervous system (motor neurons controlling skeletal muscle) and systems of afferent neurons that send sensory information into the CNS (Fletcher, 2011). The terms "neurons", "nerve cells" or "neuronal cells" are used herein interchangeably.

The term "retina" is used to describe a neuro-sensory epithelium that belongs to the CNS and is located between the eye's vitreous humour and the choroid. It captures photons which are transduced to electrical signals which are transmitted through the neuronal pathways to the visual cortical area to sense a visual picture. The retina covers the entire posterior part of the eye, apart from the area of the optic nerve. The retina also extends anteriorly to end 360 degrees at the *ora Serrata,* which is the junction between the ciliary body and the retina.

### Layers of the retina

The neural retina has ten distinct layers that are connected by synapses. There are three basic cell types: photoreceptor cells, neuronal (ganglion) cells and glial cells. The layers from the closest to the front anterior of the head towards the posterior of the head are: 1) inner limiting membrane (ILM), 2) optic nerve fiber layer (ONFL), 3) ganglion cells layer (GCL), 4) inner plexiform layer (IPL), 5) inner nuclear layer (INL), , 6) outer plexiform layer (OPL), 7) outer nuclear layer (ONL), 8) outer limiting membrane (OLM) and 9) photoreceptor layer (PL). The inner retina comprises from the ILM to the INL and the outer retina comprises from the OPL to the PL.

The ILM is the retina surface that surrounds the vitreous humor and forms a diffusion battier between the neural retina and vitreous humor. This layer is formed by the vitreal foot endings of Müller cells, a kind of macroglial cells.

The ONFL is the second innermost layer of the retina from the vitreous and contains the retinal ganglion cells (RGCs) axons that conform the optic nerve. Astrocytes, a subtype of macroglial cells are mainly located in this layer and the GCL.

The GCL comprises the RGCs and displaced amacrine cells.

The IPL is a synapse layer between RGCs and cells of the INL.

The INL comprises the cell bodies of Müller cells, bipolar cells, horizontal cells and amacrine cells.

The OPL comprises a synapse between rods and cones with bipolar and horizontal cells. The ONL contains the rod and cone's somas.

The OLM comprises the Müller cells ventricular endings.

PL contains the outer segments of photoreceptors, cones and rods, containing photosensible pigments responsible for phototransduction.

The retinal pigment epithelium does not belong to the neural retina and is the farthest retinal layer that covers the width of a single cell. It is located between the neural retina and the Bruch membrane, next to the choroid layer. It contributes to the BRB together with the endothelium of the retinal vessels. Its main functions involve preserving retinal homeostasis and recycling photopigments (Wong, J. H. C., 2022).

"Intraocular pressure" (IOP) refers to pressure exerted inside the eyes. Since pressure is a measurement of force *per* surface, the IOP is a measurement of the force exerted by the aqueous humour on the internal area of the anterior chamber of the eye. IOP is tightly regulated, and changes are often connected to the development of ocular diseases like uveitis, retinal detachment and glaucoma (Kang, J. M., & Tanna, A. P. 2021).

"Retinal ganglion cells (RGCs)" are described as neurons that transmit visual information to the visual processing centres in the central nervous system through axons, comprising the optic nerve. A wide diversity of RGCs exists, having each subtype distinct morphological features and functions; and use different connection patterns inside the visual pathway. The visual pathway consists of different relay nuclei that link the retina with the visual cortical areas (Kang, J. M., & Tanna, A. P. 2021).

"Macroglial cells" or "macroglia" provide physical support and maintain the retinal environment in homeostatic conditions, including ion, neurotransmitters and water metabolism, as well as cell debris removal. Retinal macroglia comprise astrocytes and Müller cells. They play a key role in information processing in neural circuits, retinal glucose metabolism, removing metabolic subproducts, regulating local blood flow and maintaining the BRB. They are also involved in local immune response and protect neurons from oxidative damage. Under pathological circumstances, astrocytes and Müller cells increase the expression of different proteins, such as glial fibrillary acidic protein (GFAP) (Shinozaki & Koizumi, 2021; Fernández-Albarral *et al.,* 2022). Macroglial activation is also referred as reactive macrogliosis, during which macroglial cells proliferate, expand their somas, elongate and multiply their somas, and increase the expression of a variety of proteins, such as GFAP, nestin and vimentin. Reactive macrogliosis, may have neuroprotective effects; however, its chronification and/or maladaptive function will cause BRB disruption and perpetuate the glial response due to the release of chemokines that will not only attract microglia, but also foster the migration of monocytes, macrophages, dendritic cells and T-cells. Macroglial cells have the capacity to initiate the adaptive immune response. All of the previously mentioned responses, if chronified, may develop a neuroinflammatory process that may potentially exert a neurodegenerative effect over the RGCs (Fernández-Albarral *et al.,* 2022). In advance stages of neuroinflammation there is generally also neurodegeneration.

"Macroglial response" is a reactive process involving macroglial cells comprising the defense of the CNS from damage upon recognition of several insults like trauma, ischemic damage, neuroinflammation or neurodegeneration. It can also be named reactive astrogliosis, and this process may involve the proliferation of astrocytes and Müller cells, an increase in the number of astroglial processes hypertrophy of cells, migration, and an increase in the expression of cytoskeletal proteins such as glial fibrillary acidic protein (GFAP), vimentin and nestin (Triviño *et al.,* 2006). A reactive gliosis has been associated with several retinal pathologies, comprising age-macular degeneration (AMD), glaucoma, diabetes, retinal detachment or retinitis pigmentosa.

"Microglial cells" or "microglia" are the main innate immune cells of the central nervous system. They are one of the first responders to pathological aggressions. Their function is to keep homeostasis and participate in host defense mechanisms by sensing changes in their environment, being housekeepers comprising migration to injured sites, remodelling synapses, and protecting against dangerous stimuli, comprising pathogen-associated molecular patterns (PAMPs) and damage-associated molecular patterns (DAMPs) (Kwon and Koh, 2020). In the retina, they are mainly located in the ONFL, GCL, IPL and OPL.

"Microglial response" refers to a reactive process in which microglial cells respond to cytokines and other signalling mediators from acute inflammation. Microglia switch from a surveillant and ramified state to an activated and phagocytic one. This process involves the release of pro-inflammatory cytokines and phagocytosis of damaged tissue. The activation of microglia for long periods can result to sustained production of cytokines and other neurotoxic molecules that can lead to long-term neuroinflammation and potentially neurodegeneration (Liu and Hong, 2003). Microglial cells express the ionized calcium-binding adapter molecule 1 (lba-1), a marker that has been extensively used to identify this cell type by immunohistochemical techniques. Under physiological conditions, microglia can be found in a quiescent radial form (RD morphotype). Under pathological conditions, microglia acquire a reactive state and can migrate either across the different retinal layers, acquiring a vertical morphotype (VT) with processes being perpendicular to the layers and using the Müller cells as a scaffold; or move along a single retinal layer, acquiring a horizontal morphotype (HZ), with processes being parallel to the retinal layers. Activated microglia may release pro-inflammatory cytokines (TNF-α, IL-1β, IL-6 and IL-12) that, if not regulated, may directly induce RGCs apoptotic death. Indeed, an increase in inflammatory mediators, such as IL-1β, IL-6, IL-12, IFN-y, VEGF and TNF-α, has been reported in the aqueous humor, serum and retina of glaucoma eyes both in patients and in different animal models (Fernández-Albarral *et al.,* 2021).

The term "treatment" as used herein refers to the prophylactic and/or therapeutic treatment.
The term "therapeutic treatment" as used herein refers to bringing a body from a pathological state or disease back to its normal, healthy state. Specifically, unless otherwise indicated, includes the amelioration, cure, and/or maintenance of a cure (i.e., the prevention or delay of relapse) of a disease or disorder. Treatment after a disorder has started aims to reduce, alleviate, ameliorate or altogether eliminate the disorder, and/or its associated symptoms, to prevent it from becoming worse, to slow the rate of progression, or to prevent the disorder from re-occurring once it has been initially eliminated (i.e., to prevent a relapse). It is noted that, this term as used herein is not understood to include the term "prophylactic treatment" as defined herein.

"Intraocular pressure-lowering treatments" are treatments that cause a reduction in the intraocular pressure.

The term "prophylactic treatment" or "preventive treatment" as used herein refers to preventing a pathological state. It is noted that, this term as used herein is not understood to include the term "therapeutic treatment" as defined herein.

The term "therapeutically effective amount" as used herein refers to an amount that is effective, upon single or multiple dose administration to a subject (such as a human patient) in the prophylactic or therapeutic treatment of a disease, disorder or pathological condition.

The term "subject" as used herein refers to a mammalian subject. Preferably, it is selected from a human, companion animal, non-domestic livestock or zoo animal. For example, the subject may be selected from a human, mouse, rat, dog, cat, cow, pig, sheep, horse, bear, and so on. In a preferred embodiment, said mammalian subject is a human subject.

The term "combination" or "combination therapy" as used throughout the specification, is meant to encompass the administration of the referred therapeutic agents to a subject suffering from a disease, disorder or pathological condition, in the same or separate pharmaceutical formulations, and at the same time or at different times. If the therapeutic agents are administered at different times they should be administered sufficiently close in time to provide for the potentiating or synergistic response to occur.

The term "single agent" as used herein relates to the use of an active ingredient sufficiently separate in time from another active ingredient to prevent for the potentiating or synergistic response to occur. More specifically, the use as "single agent" does not encompass the use as a "combination therapy".

The term "active ingredient" as used herein refers to any component that provides pharmacological activity.

The terms "agent" and "drug" are used herein interchangeably.

The term "medicament" describes a drug or combination of drugs that have the ability to treat or prevent diseases, disorders or conditions in subjects or that they can be used in subjects with the aim of restoring, correcting or modifying the physiological functions performing a pharmacological, immunological or metabolic function.

The terms "medicament" and "medication" are used herein interchangeably.

### Detailed description

The first aspect of the invention relates to a DHP-I inhibitor for use as anti-inflammatory and/or neuroprotective medicament or for use as neuroprotectant in a method of treating a mammalian subject.

In a second aspect, the present invention provides a DHP-I inhibitor for use in a method for the prophylactic and/or therapeutic treatment of a neuroinflammatory disease in a mammalian subject.

In a related aspect, it refers to a method for the prophylactic and/or therapeutic treatment of a neuroinflammatory disease, wherein this method comprises administering to a subject in need of such treatment therapeutically effective amount of a DHP-I inhibitor.

It further provides the use of a DHP-I inhibitor in the manufacture of a medicament for the prophylactic and/or therapeutic treatment of a neuroinflammatory disease in a mammalian subject.

A "neuroinflammatory disease" is a neurological or mental disease of the central nervous system (CNS) and/or the peripheral nervous system (PNS), which pathogenesis involves primary or secondary neuroinflammation and may evolve into neurodegeneration.

In some embodiments of any thereof, it relates to a DHP-I inhibitor in the prophylactic treatment of neurodegeneration. In preferred embodiments of any thereof, the neuroinflammatory disease is a neuroinflammatory and/or neurodegenerative disease.

In some embodiments, the neuroinflammatory disease is a mental disorder. Interestingly, neuroinflammation has been described to occur in mental diseases or disorders, such as schizophrenia (Vallée A. 2022) or depression (Nettis MA, Pariante CM 2020).

In other embodiments, the neuroinflammatory disease is a neurological disease affecting the CNS and/or the PNS. Disorders of the PNS include but are not limited to acute motor axonal neuropathy, Bell's palsy, botulism, brachial plexus, carpal tunnel syndrome, cubital tunnel syndrome, Charcot-Marie-Tooth disease types 1A, 1B and 1X, cisplatin neuropathy, chronic inflammatory demyelinating polyradiculoneuropathy, diabetic neuropathy, diphtheritic neuropathy, familial amyloid neuropathy, Guillain-Barré syndrome, hepatitis C, human immunodeficiency virus (HIV) infection, Lambert-Eaton syndrome, leprosy, Lyme disease, lumbosacral neuropathies, lumbosacral plexus, neuropathy with IgM1 anti-myelin-associated glycoprotein, nutritional neuropathies, oxaliplatin neuropathy, pyridoxine neuropathy, postherpetic neuralgia, radial neuropathies, Refsum's disease, trigeminal neuralgia, thoracic outlet syndrome and uremic neuropathy (Vitrikas and Hurst, 2022). In some embodiments, the neuroinflammatory disease is a neurological disorder other than a neuropathic peripheral disorder.

Neurodegenerative diseases represent a large number of neurological disorders, which can affect specific subgroups of neurons in determinate regions of the CNS and/or PNS. This group comprises but is not limited to Alzheimer's disease, Parkinson's disease, amyotrophic lateral sclerosis, multiple sclerosis and ocular neurodegenerative diseases, such as glaucoma (Kwon and Koh, 2020). Neurodegenerative diseases are neuroinflammatory diseases. The common characteristic among all neurodegenerative diseases is neuroinflammation, that leads to neurodegeneration, meaning neuronal death. Another feature that neurodegenerative diseases share is the selective susceptibility of brain regions and protein aggregation. Although chronic inflammation was thought to be the result of protein aggregation, it can also trigger the formation of aggregates at the earliest stages in some diseases such as Alzheimer (Hammond *et al.,* 2019). In some embodiments, the neuroinflammatory disease is an advanced stage neuroinflammatory disease where there is also neurodegeneration. In other embodiments, said neuroinflammatory disease is an early stage neuroinflammatory disease. In preferred embodiments, the early stage neuroinflammatory disease is characterized by absence of neurodegeneration or by normal levels of neuronal death, such as those occurring in healthy human adults.

In preferred embodiments, the neuroinflammatory disease is selected from the group consisting of Alzheimer's disease (AD), Parkinson's disease (PD), amyotrophic lateral sclerosis (ALS), multiple sclerosis (MS), ocular neurodegenerative diseases, primary tauopathies, frontotemporal dementia (FTD), synucleinopathies (e.g., Lewy body dementia [LBD] and multisystem atrophy [MSA]), Huntington's disease (HD) and related polyglutamine (polyQ) diseases (including spinocerebellar ataxias [SCA]), prion disease (PrD), traumatic brain injury (TBI), chronic traumatic encephalopathy (CTE), stroke, migrane, spinal cord injury (SCI) and autoimmune encephalitides, such as systemic lupus erythematosus, Hashimoto encephalopathy, Sydenham chorea, Behçhet disease, Rasmussen encephalitis and limbic encephalitis epilepsy (see for instance, Wilson et al., 2023).

Neuroinflammation may be caused by endogenous factors (e.g., protein aggregates), environmental factors (e.g., systematic infection, gut commensal dysbiosis, aging, diet), and/ or genetic susceptibility (e.g., progranulin (PGRN) mutations, apolipoprotein E4 (APOE4) mutations), see for instance Zhang et al., 2023. Accordingly, neuroinflammatory diseases may in some embodiments be classified according to their aetiology.

Examples of neuroinflammatory diseases caused by endogenous factors are neurodegenerative diseases where there is protein aggregation such as Alzheimer's disease or Parkinson's disease. Examples of neuroinflammatory diseases induced by environmental factors are Traumatic brain injury (TBI), chronic traumatic encephalopathy (CTE) and spinal cord injury (SCI). Finally examples of neuroinflammatory diseases where genetic susceptibility is involved are Alzheimer's disease (APOE, TREM2), Parkinson's disease (LRRK2, glucocerebrosidase, PRKN), amyotrophic lateral sclerosis.

In some embodiments, the neuroinflammatory disease is an ocular neurodegenerative disease. Neurodegenerative processes may involve the CNS, including the retina and visual pathway, being the main mechanism the anterograde (from the retina towards the rest of the visual pathway) and retrograde (from any point of the visual pathway to the retina) neurodegeneration involving RGCs death Retinal immune homeostasis is maintained by the immune system and the immune response function. However, various genetic, metabolic and environmental factors can impair retinal homeostasis, initiating inflammatory pathways. Prolonged inflammation may play a role in the initiation and development of ocular neurodegenerative diseases (Kaur and Singh, 2023). In some embodiments, the ocular neurodegenerative disease is selected from the group consisting of glaucoma, age-related macular degeneration (AMD), diabetic retinopathy (DR) retinitis pigmentosa (RP) and neuromyelitis optica spectrum disorders (NMOSD) .

Age-related macular degeneration (AMD) is an ocular disorder that affects the macula, which is the central area of the retina, and causes a loss of distinct vision. AMD can be classified into a dry form (with an incidence of 80%) and a wet form (with an incidence of 20%). Diabetic retinopathy (DR) is a complication of diabetes impairing vision, caused by the injury of the blood vessels of the retina. This disease affects both type 1 and type 2 diabetes patients. DR is divided into non-proliferative diabetic retinopathy and proliferative or advanced diabetic retinopathy. Retinitis pigmentosa (RP) is a group of inherited of retinal dystrophies that cause a progressive loss of the photoreceptors and retinal pigment epithelium leading to blindness.

In a preferred embodiment, the neuroinflammatory disease is glaucoma. Glaucoma is a group of optic neuropathies featured by progressive degeneration of RGCs which leads to visual loss. RGCs pass on the visual information to the brain via axons, which form the optic nerve. The optic disc is the site where retinal ganglion cells axons join, make a 90° turn, and pass through the sclera and lamina cribosa to exit the globe as the optic nerve. In the center of the optic disc there is a depression named the optic cup. In glaucoma, the injury of the lamina cribosa and loss of RGC axons leads to the progressive enlargement of the optic cup (Kang and Tanna, 2021).

Glaucoma can be divided in two groups: open-angle glaucoma (OAG) and angle-closure glaucoma (ACG). This classification describes the anatomic status of the anterior chamber iridocorneal angle and each type can be further subclassified into primary or secondary disease. While primary glaucoma is characterized by the absence of other clinically identifiable ocular or systemic disorders that can be attributed exclusively to glaucoma, secondary glaucoma are featured by its presence (Kang and Tanna, 2021). Another type of glaucoma are primary childhood glaucoma (PCG), which are rare and include: primary congenital glaucoma, late-onset childhood open angle glaucoma with onset from more than two years of age to puberty and secondary childhood glaucoma that can be divided into: glaucoma associated with non-acquired ocular anomalies, glaucoma associated with non-acquired systemic disease or syndrome, glaucoma associated with acquired condition and glaucoma following childhood cataract surgery (European Glaucoma Society Terminology and Guidelines for Glaucoma, 2021).

### Open angle glaucoma

### Primary open angle glaucoma

Primary open angle glaucoma (POAG) is a chronic and progressive irreversible eye disorder which causes optic nerve rim and retinal nerve fiber layer loss with related visual field impairment. Among risk factors, older age, elevated IOP, ethnicity, family history of glaucoma, disc haemorrhage, pseudoexfoliation and myopia have been reported for OAG.

It is thought that in POAG high IOP levels are not well tolerated by the individual eye and lead to the deformation of the lamina cribrosa. Both the mechanical pressure exerted directly onto RGCS and this deformation may lead to the apoptosis of RGC, with vascular factors being involved as well.

POAG has been further divided into "high pressure" and "normal pressure" disease. In "normal pressure" or "normal tension" glaucoma, the IOP is normal or even within the lower normal range. Since glaucoma with lower pressure levels affects a substantial number of patients (up to 30% in the western hemisphere and up to 90% up to 90% in the far east), other risk factors than IOP have great importance. Glaucoma with lower levels of IOP may be more prevalent in women with vascular dysregulation (including migraine and Raynaud syndrome), as well as women undergoing anticonceptive hormonal treatments. The terms "normal pressure glaucoma", "normal tension glaucoma" and "normotensive glaucoma" are used herein interchangeably.

### Secondary open angle glaucoma

Secondary open angle glaucoma (OAG) is a heterogeneous group of conditions with high IOP being the leading pathological factor. OAG can be further classified into secondary open angle glaucoma caused by ocular disease, secondary open angle glaucoma due to ocular trauma, Iatrogenic secondary open angle glaucoma and secondary open angle glaucoma caused by extraocular disease. Among the first type there are pseudoexfoliative or exfoliative glaucoma (PXFG), pigmentary glaucoma (PG), lens-induced open angle glaucoma, glaucoma associated with intraocular haemorrhage, uveitic glaucoma, neovascular glaucoma and glaucoma due to intraocular tumors. Regarding the Iatrogenic secondary open angle glaucoma, the subtypes glaucoma due to corticosteroid treatment, secondary open angle glaucoma due to ocular surgery and laser and glaucoma associated with vitreoretinal surgery are found.

### Angle closure

Angle closure can be defined as the presence of adhesions of the iris to the trabecular meshwork and/or ocular hypertension without glaucomatous optic nerve damage. Although it is chronic and asymptomatic, it is considered clinically relevant when the iridotrabecular contact (ITC) is greater than 180 degrees. Angle closure may produce an increase in IOP leading to glaucomatous optic neuropathy. The mechanisms that cause angle closure may be described by the anatomical factor that provokes the obstruction of the aqueous flow: the iris, ciliary body, lens or causes behind the lens. Different mechanisms can co-exist and vary with race.

### Primary angle closure (PAC)

There are three stages of PAC: primary angle closure suspect (PACS), primary angle closure (PAC) and primary angle closure glaucoma (PACG). Angle closure may alter the aqueous outflow through the obstruction or damage of the trabecular meshwork. The trabecular meshwork is an area of tissue located in the base of the cornea, near the ciliary body. Its function is to drain the aqueous humor from the eye through the anterior chamber. The main risk factors for developing PAC are older age, female sex, family history, hypermetropia and race.

### Secondary angle closure

Secondary angle closure originates from many causes. The clinical signs are distinct depending on the underlying condition. Within secondary angle closure, there are many subtypes: secondary angle closure with pupillary block, secondary angle closure with anterior 'pulling' mechanism synechial closure without pupillary block (including neovascular glaucoma, iridocorneal endothelial syndrome, and epithelial and fibrous ingrowth after anterior segment surgery or penetrating trauma), secondary angle closure with posterior 'pushing' mechanism without pupillary block (including aqueous misdirection or malignant glaucoma; iris and ciliary body cysts, intraocular tumors, Silicon oil or other tamponading fluids or gas implanted in the vitreous cavity, uveal effusion, retinopathy of prematurity and congenital anomalies that can be associated with secondary angle closure glaucoma).

In some embodiments, the glaucoma is open angle glaucoma, such as primary open angle glaucoma or secondary open angle glaucoma. In other embodiments, the glaucoma is angle-closure glaucoma (ACG), such as primary angle closure glaucoma or secondary angle closure glaucoma. In other embodiments, optionally in combination with any of the above, the glaucoma is (i) normotensive glaucoma or (ii) high pressure glaucoma. In preferred embodiments, the glaucoma is high pressure glaucoma resistant to intraocular pression (IOP)-lowering treatment, such as resistant to prostaglandin analogs. IOP-lowering treatments are described in further detail herein below.

In some embodiments, the DHP-I inhibitor for use as defined herein results in one or more of the following effects:
a) a prevention and/or decrease of neurodegeneration or the loss of neuronal cells, and/or
b) a prevention and/or decrease of glia-mediated neuroinflammatory response, such as by reducing the inflammatory response of microglial and/or macroglial cells.

In some embodiments, the DHP-I inhibitor for use as described herein presents at least a 60%, 70%, 80%, 90%, 100%, 110%, 120%, 130%, 140% or 150% of the biological activity of cilastatin in terms of
a) a prevention and/or decrease of neurodegeneration or the loss of neuronal cells, and/or
b) a prevention and/or decrease of glia-mediated neuroinflammatory response, such as by reducing the inflammatory response of microglial and/or macroglial cells.

The loss of neuronal cells or "neuronal loss" refers to the nerve cells, neurons or neuronal cells death. The terms "loss", "death", "reduction" or "destruction" are used herein interchangeably. The mechanism of neuronal cell death can be through apoptosis, necrosis, necroptosis, autophagy, parthanatosis, mitochondrial permeability transition pore, ferroptosis, oncosis, pyroptosis or autolysis (Goel *et al.,* 2022, Fricker M *et al.,* 2018).

Glaucoma is characterized by the degeneration or irreversible death of RGCs. There have been reported several phenotypic markers of RGCs, notably Brain-specific homeobox/POU domain protein 3A (Brn3a)and RNA-binding protein with multiple splicing (RBPMS) (Galindo-Romero *et al.,* 2011). Brn3a also named POU domain, class 4, transcription factor 1 (Pouf4f1) is a multifunctional transcription factor that acts by binding to sequences related to the consensus octamer motif 5'-ATGCAAAT-3' in the regulatory regions of its target genes (Budhram-Mahadeo *et al.,* 1996). It regulates the expression of specific genes involved in the differentiation and survival within a subset of neuronal lineages. Brn3a also induces neuronal process outgrowth and the coordinate expression of genes encoding synaptic proteins (Smith *et al.,* 1997). It triggers BCL2 expression and prevents neuronal cells from undergoing apoptosis. The expression of Brn3a can be used as a marker to quantify RGCs in healthy and injured retina (Nadal-Nicolás *et al.,* 2009).

In some embodiments, the treatment with the DHP-I inhibitor prevents, decreases or reverses the Brn3a expression reduction in at least one region or subregion of the retina, preferably in two, three, four, five or more subregions of the retina, more preferably in the total retina, in the context of primary neuroinflammation (e.g., CONTRA eye) or secondary neuroinflammation (e.g., OHT eye). In some embodiments, optionally in combination with any of the above, the treatment with the DHP-I inhibitor prevents, decreases or reverses Brn3a expression reduction in the regions closer to the optic disc, namely one or more of N-C, N-I, C-C and C-I (see Fig.7).

The retina can be divided according to its spatial organization all along the X axis in three regions: Nasal (N), Central (C) and Temporal (T); and all along the Y axis in three other regions: Superior (S), Central (C) and Inferior (I). The combination of the X and Y axis regions results in 9 retinal subregions, namely the nasal-superior (N-S), nasal-central (N-C), the nasal-inferior (N-I), the central-superior (C-S), the central-central (C-C), the central-inferior (C-I), the temporal-superior (T-S), the temporal-central (T-C) and the temporal-inferior (T-I) subregions, see Fig. 7.

Macroglial cells, namely astrocytes and Müller cells, are characterized by the expression of GFAP. This protein has also been found in non-myelinating Schwann cells in the PNS and the glia cells of the enteric nervous system (ENS). The upregulation of GFAP can reveal alterations in the molecular expression and morphology of astrocytes and Müller cells, indicating the severity of reactive macrogliosis and a hallmark of CNS pathology.

In some embodiments, optionally in combination with any of the above, the treatment with the DHP-I inhibitor prevents, decreases or reverses the increase of GFAP expression in at least one region or subregion of the retina, preferably in two, three, four, five or more subregions of the retina, more preferably in the total retina, in the context of primary neuroinflammation (e.g., CONTRA eye) or secondary neuroinflammation (e.g., OHT eye). In some embodiments, the treatment with DHP-I inhibitor prevents, decreases or reverses the increase of GFAP expression in at least one region or subregion, preferably in all regions or subregions, of the inner retina, more preferably in one or more of ONFL/GCL, IPL and INL. In other embodiments, the treatment with DHP-I prevents, decreases or reverses the increase of GFAP expression in at least one region or subregion, preferably in all regions or subregions, of the outer retina, more preferably in one or more of OPL, ONL and PL.

In some embodiments, optionally in combination with any of the above, the treatment with the DHP-I inhibitor prevents, decreases or reverses the increase of GFAP expression in the areas closer to the optic disc. In other embodiments, optionally in combination with any of the above, the treatment with the DHP-I inhibitor prevents, decreases or reverses the increase of GFAP expression in at least one, preferably one, two, three, four or more of the retinal layers, such as in all the retinal layers.

As previously mentioned, Iba-1 is a marker that identifies microglial cells and its expression is upregulated in activated microglia, for instance following facial nerve axotomy, ischemia, and several brain diseases (von Ertzen *et al.,* 1998), highlighting its implication in the activated phenotypes of microglia.

In some embodiments, optionally in combination with any of the above, the treatment with the DHP-I inhibitor prevents, decreases or reverses the increase of Iba-1 expression in at least one region or subregion of the retina, preferably in two, three, four, five or more subregions of the retina, more preferably in the total retina, in the context of primary neuroinflammation (e.g., CONTRA eye) or secondary neuroinflammation (e.g., OHT eye). In some embodiments, the treatment with DHP-I inhibitor prevents, decreases or reverses the increase of Iba-1 expression in at least one region or subregion, preferably in all regions or subregions, of the inner retina, more preferably in one or more of ONFL/GCL, IPL and INL. In other embodiments, the treatment with DHP-I prevents, decreases or reverses the increase of Iba-1 expression in at least one region or subregion, preferably in all regions or subregions, of the outer retina more preferably in one or more of OPL, ONL and PL.

In some embodiments, optionally in combination with any of the above, the treatment with the DHP-I inhibitor prevents, decreases or reverses the increase of Iba-1 expression in the areas closer to the optic disc. In other embodiments, optionally in combination with any of the above, the treatment with the DHP-I inhibitor prevents, decreases or reverses the increase of the Iba-1⁺ cells in at least one, preferably one, two, three, four or more of the retinal layers, such as in all the retinal layers.

### Route of administration

The DHP-I inhibitor for use according to the first or second aspects is typically formulated as a pharmaceutical composition to be compatible with its intended route of administration. Methods to accomplish the administration are known to those of ordinary skill in the art. This includes, for example, injection or infusion, by parenteral routes such as intravascular (e.g., intravenous or intraarterial), subcutaneous, intramuscular, intraperitoneal, intraventricular, intraepidural, or others as well as oral, sublingual, nasal, ophthalmic, intrathecal, intracranial, rectal, transdermal or topical. Sustained release administration is also specifically contemplated, e.g., as depot injections or erodible implants. Localized delivery is particularly contemplated, e.g., as delivery via a catheter to one or more arteries or a vessel supplying a localized site of interest.

### Parenteral drug delivery

In some embodiments, the DHP-I inhibitor is administered by the parenteral route. Preferably, the DHP-I inhibitor is administered by the intramuscular, intraperitoneal or intravascular route of administration. More preferably, said composition is in a form suitable for intravascular administration, e.g., by the intravenous route. Administration by the intravascular route is carried out using devices well known in the art, which are used to administer fluids from a container to a patient's vascular system through a needle or catheter inserted into a vessel, such as a vein. The device may include the needle or catheter, tubing, a flow regulator, a drip chamber, an infusion line filter, an I.V. set stopcock, fluid delivery tubing, connectors between parts of the set, a side tube with a cap to serve as an injection site, and a hollow spike to penetrate and connect the tubing to an I.V. bag or other infusion fluid container.

### Ophthalmic drug delivery

The routes for ocular drug administration include topical, systemic, intraocular and periocular administration. In a particular embodiment, the DHP-I inhibitor is administered locally in the eye, such as by topical, intraocular or periocular administration. Further embodiments and details for each of these administration routes are provided herein below.

### - Topical drug delivery

Topical drug delivery is normally in the form of eye drops. Topical administration is normally used to treat diseases affecting the anterior segment of the eye. It delivers the drug into the lower precorneal pocket of the eye. This route of administration is patient compliant, safe, easy and non-invasive. One major disadvantage is that 95% of the drug is lost owing to tear turnover and reflux blinking. Examples of topical eye delivery systems are suspensions, ointments, hydrogels and drug-loaded contact lenses.

### - Systemic drug delivery

Systemic route of administration is used to reach the posterior segment of the eye, for instance through intravenous and oral administration. Drugs administered by systemic route reach through the blood vessels present in the posterior segment of the eye. However, drug penetration can be limited by blood-retinal barrier and blood-aqueous barrier. The permeation of drugs can be impaired into the aqueous humor due to two cell layers that form the blood-aqueous barrier. Although oral drug delivery is used to treat chronic retinal diseases, drug penetration into the ocular tissues may be limited.

### - Intraocular drug delivery

Intraocular delivery allows the administration of the drug close to the target tissue. It normally needs high drug concentration, which may entail significant adverse reactions. Intraocular drug delivery includes for instance intracameral and intravitreal injections. Intracameral injections refer to the injection of the drug into the anterior segment of the eye or in the vitreous cavity. Intravitreal injections and implants deliver the drug into the vitreous close to the retina at the posterior part of the eye. Drugs that have been injected into the vitreous may not be uniformly distributed and large molecule penetration can be limited. High lOP can be a consequence of intravitreal administration.

### - Periocular drug delivery

Periocular drug delivery route can reach various layers of the ocular tissue. It is considered to be safer to reach the retina and other remote tissues. Periocular route includes subconjunctival, retrobulbar, peribulbar, subtenon, posterior juxtascleral, transscleral and suprachoroidal.

For subconjunctival drug delivery, drugs are inserted, injected or implanted below the conjunctiva. This route of administration is effective delivering drugs to anterior and posterior segments of the eyes. Retrobulbar injection delivers drugs into the muscle cone in the back of the eye globe. This kind of injection poses a risk for damaging the optic nerve. With this route, high local concentration of drugs is achieved. Peribulbar injection is simpler than retrobulbar injection, being the first a safer route of administration. It can be, however, less effective than retrobulbar injection with an increased risk of globe perforation. For subtenon drug delivery, drugs are injected into the Tenon's capsule in the upper section of the eye. It is fairly non-invasive, with fewer complications and a long-term contact of the drug with the sclera is achieved. This route of delivery is used to administer anaesthesia in ocular surgery. For posterior juxtascleral delivery, drugs are supplied directly to the outer surface of the sclera. This route of administration is safe for the delivery of depot formulations. It decreases the risk of endophthalmitis and intraocular damage. On the other hand, posterior juxtascleral delivery is more complex than other periocular routes. Transscleral route is accessible, allowing the permeability of small and large molecules and the administration of higher doses with reduced systemic side effects in comparison to intravitreal route. One of the limitations of the use of this route is that the sclera and choroid can act as a barrier to the drug reaching the retina. Finally, suprachoroidal administration delivers the drug to the choroid and the retina. Although it is suitable for small molecules and drugs reach low systemic levels, it may not be suitable for sustained released formulations due to fast clearance (Ahmed *et al.,* 2023, Dubald *et al.,* 2018, Rafiei *et al.,* 2020).

In some embodiments, the DHP-I inhibitor is administered locally in the eye by topical administration. In other embodiments, the DHP-I inhibitor is administered locally in the eye by intraocular administration, preferably selected from intravitreal and intracameral administration. In still further embodiments, the DHP-I inhibitor is administered locally in the eye by periocular administration, preferably selected from subconjunctival, retrobulbar, peribulbar, subtenon, posterior juxstascleral, transscleral and suprachoroidal administration.

### Administration schedule and dosage

The DHP-I inhibitor can be administered a single time. It may also be administered regularly throughout the course of the method of treatment, for example, one, two, three, four, or more times a day, every other day, weekly, bi-weekly, every three weeks or monthly. In a particular embodiment, the DHP-I inhibitor is administered daily, preferably three or four times per day, from the diagnosis of glaucoma or suspected glaucoma until medical criteria. The DHP-I inhibitor may also be administered continuously to the subject (e.g., intravenously or by release from an implant, pump, sustained release formulation, etc.).

The dosage to be administered can depend on multiple factors, including the type and severity of the disease and/or on the characteristics of the subject, such as general health, age, sex, body weight and tolerance to drugs and should be adjusted, as needed, according to individual need and professional judgment. The dosage may also vary depending upon factors, such as route of administration, treatment regime, target site, or other therapies administered. The skilled artisan will be able to determine appropriate doses depending on these and other factors. A prophylactic or therapeutically effective amount may include, but is not limited to, dosage ranges of about 0.1 mg/kg to about 100 mg/kg of body weight, preferably about 10 mg/kg to about 50 mg/kg, more preferably, about 20 mg/kg to about 40 mg/kg.

In a particular embodiment, the DHP-I inhibitor (e.g., cilastatin) is administered by the parenteral route of administration (preferably intravenously) at a dosage of about 10 to about 100 mg/kg of body weight, preferably of about 15 to about 60 mg/kg of body weight, more preferably of about 25 to about 40 mg/kg of body weight for a human adult.

Preferred dosage and administration schedules of the DHPI-inhibitor (e.g., cilastatin) for a human adult are daily doses from 1 g to 4 g by the parenteral route (preferably intravenously), such as by giving 250 mg every 6h, 500 mg every 6h or 8h, or 1 g every 6h or 8h.

The DHP-I inhibitor may be administered alone (as a single agent) or in combination with another treatment. Preferably, said other treatment is one or more treatments for treating glaucoma, such as a treatment for reducing IOP or for treating pupillary block. These are further defined herein below.

Topical IOP-lowering medication or laser trabeculoplasty are first-line treatments against glaucoma (Gazzard *et al.,* 2019). The pupillary block is typically also treated in primary angle-closure disease. To this end, iridotomy or lens extraction is needed in addition to IOP-lowering medications.

The treatment of severe, acute IOP increase is managed with topical and oral medications, at times systemic hyperosmotic agents, e.g. mannitol or glycerin, can also be used. In the case that the acute IOP increase is caused by pupillary block, iridotomy or lens extraction procedures are typically performed. Acute angle-closure crisis is normally unilateral, but 50% of contralateral eyes may develop an acute crisis in the five following years (Prum *et al.,* 2016). The primary objective of lens extraction in that case is to open the iridocorneal angle by substituting the large, natural lens, with a thin, artificial lens.

Intraocular pression (IOP)-lowering treatments comprise medical therapy (IOP-lowering drugs), laser surgery (laser trabeculoplasty and laser peripheral iridotomy), incisional surgery (trabeculectomy, trabeculectomy and goniotomy, non-penetrating glaucoma surgery, long-tube glaucoma drainage devices, additional/alternative surgical techniques, cataract and glaucoma surgery) and cyclodestructive procedures. The later include *inter alia* lasers (transscleral diode cyclophotocoagulation, micropulse laser cyclophotocoagulation and direct and endoscopic cyclophotocoagulation), ultrasound (high intensity focused ultrasound circular cyclocoagulation) and cryoprobe.

Medical therapy for treating glaucoma aims to lower lOP through the reduction of aqueous humor production or improving outflow. In some embodiments, the DHP-I inhibitor or a composition comprising thereof is administered in combination with one or more IOP-lowering drugs. Preferably, the IOP-lowering drugs are selected from one or more of the group consisting of: i) prostaglandin analogs (PGA) such as latanoprost, travoprost, bimatoprost, tafluprost, latanoprostene bunod, omidenepag isopropyl, and unoprostone isopropyl, ii) α-antagonists of non-adrenergic receptors (AAAs) such as bunazosin hydrochloride and dapiprazole, iii) cholinergic drugs (miotics) such as pilocarpine hydrochloride, iv) Rho kinase inhibitors (RKIs) such as netarsudil and ripasudil mesylate, v) β-adrenergic (BB) such as timolol, betaxolol, betaxolol, carteolol, levobunolol, levobetaxolol hydrochloride and nipradilol, vi) α-adrenergic receptors (AAs) such as apraclonidine, brimonidine, clonidine, phenylephrine hydrochloride and dipivefrine hydrochloride and vii) carbonic anhydrase inhibitors (CA inhibitors) such as dorzolamide, brinzolamide, acetazolamide and methazolamide.

In some embodiments, the DHP-I inhibitor and said other drug or treatment as described herein are administered simultaneously and preferably the DHP-I inhibitor and said other drug are part of the same pharmaceutical composition. In other embodiments, the DHP-I inhibitor and said other drug or treatment as described herein are administered separately. In such instances, one can administer both therapeutic agents within a given time interval, such as about 12-24 hours of each other and, more preferably, within about 6-12 hours of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations. In other situations, it might be desirable to reduce the time between administration, administering both therapeutic agents within seconds or minutes to hours, such as within about 4 hours or 6 hours from each other, more preferably within about 1 or 3 hours.

In some embodiments, optionally in combination with any of the above, the DHP-I inhibitor is administered in combination with laser surgery, such as laser trabeculoplasty or laser peripheral iridotomy. In other embodiments, optionally in combination with any of the above, the DHP-I inhibitor is administered in combination with cyclodestructive procedures. In further embodiments, optionally in combination with any of the above, the DHP-I inhibitor is administered in combination with incisional surgery. Incisional surgery includes trabeculectomy, trabeculectomy and goniotomy, non-penetrating glaucoma surgery, long-tube glaucoma drainage devices, additional/alternative surgical techniques, antifibrotics agents used after incisional surgery and cataract and glaucoma surgery.

### Pharmaceutical composition

In a third aspect, the present invention also provides a pharmaceutical composition comprising a DHP-I inhibitor (e.g., cilastatin) and a pharmaceutically acceptable excipient, for use according to the first or second aspects. In preferred embodiments, the pharmaceutical composition is according to the fifth or sixth aspects.

Pharmaceutically acceptable excipients include, but are not limited to a carrier or diluent, such as a gum, a starch (e.g. corn starch, pregeletanized starch), a sugar (e.g. lactose, mannitol, sucrose, dextrose), a cellulosic material (e.g. microcrystaline cellulose), an acrylate (e.g. polymethylacrylate), calcium carbonate, magnesium oxide, talc, or mixtures thereof; a binder (e.g. acacia, cornstarch, gelatin, carbomer, ethyl cellulose, guar gum, hydroxypropyl cellulose, hydroxypropyl methyl cellulose, povidone); a disintegrating agent (e.g. cornstarch, potato starch, alginic acid, silicon dioxide, croscarmelose sodium, crospovidone, guar gum, sodium starch glycolate), a buffer (e.g. Tris-HCl, acetate, phosphate, bicarbonate) of various pH and ionic strength; and additive such as albumin or gelatin to prevent absorption to surfaces; a detergent (e.g. Tween 20, Tween 80, Pluronic F68, bile acid salts); a protease inhibitor; a surfactant (e.g. sodium lauryl sulfate); a permeation enhancer; a solubilizing agent (e.g. glycerol, polyethylene glycerol); an antioxidants (e.g. ascorbic acid, sodium metabisulfite, butylated hydroxyanisole); a stabilizer (e.g. hydroxypropyl cellulose, hydroxypropylmethyl cellulose); a viscosity increasing agent (e.g. carbomer, colloidal silicon dioxide, ethyl cellulose, guar gum); a sweetener (e.g. aspartame, citric acid); a preservative (e.g. thimerosal, benzyl alcohol, parabens); a lubricant (e.g. stearic acid, magnesium stearate, polyethylene glycol, sodium lauryl sulfate); a flow-aid (e.g. colloidal silicon dioxide), a plasticizer (e.g. diethyl phthalate, triethyl citrate); an emulsifier (e.g. carbomer, hydroxypropyl cellulose, sodium lauryl sulfate); a polymer coating (e.g. poloxamers or poioxamines); a coating and film forming agent (e.g. ethyl cellulose, acrylates, polymethacrylates); a pharmaceutically acceptable carrier for liquid formulations, such as an aqueous (water, alcoholic/aqueous solution, emulsion or suspension, including saline and buffered media) or non-aqueous (e.g., propylene glycol, polyethylene glycol, and injectable organic esters, such as ethyl oleate) solution, suspension, emulsion or oil; and a parenteral vehicle (e.g., for subcutaneous, intravenous, intraarterial, or intramuscular injection), including but not limited to, water, oils, saline solution, Ringer's dextrose, aqueous dextrose and other sugar solutions. A pharmaceutically acceptable excipient also includes excipients for nanoencapsulation purposes, such as a cationic polyelectrolyte (e.g. gelatin and an anionic polyelectrolyte (e.g. arabic gum).

In some embodiments, said composition is an aqueous composition, preferably a stable aqueous composition. As used herein, a "stable composition" may refer to a formulation in which the active ingredient therein essentially retains its physical stability and/or chemical stability and/or biological activity upon storage.

Said aqueous composition conventionally comprises water or an aqueous solution. Typically, the DHP-I inhibitor (e.g., cilastatin) is formulated, preferably as single agent or single active ingredient, in a buffered solution adjusted in the pH range of 6.5-8.5. Suitable diluents include but are not limited to 0.9% sodium chloride injection, 5% or 10% dextrose injection; 5% dextrose and 0.9% sodium chloride injection; 5% dextrose injection with 0.225% or 0.45% saline solution; 5% dextrose injection with 0.15% potassium chloride solution; mannitol 5% and 10%. In a preferred embodiment, said diluent is 0.9% sodium chloride injection; or 5% dextrose injection.

Some embodiments of such compositions may be provided by lyophilised formulations. Said lyophilised formulations can be reconstituted and diluted to give a composition in the form of a solution ready for intravascular injection. By way of illustration, but not as limitations, embodiments of lyophilised formulations are reconstituted with a volume of a diluent as described above. Preferably, said lyophilised formulations are presented in single dose, such as 250 mg or 500 mg of the DHP-I inhibitor (e.g., cilastatin) containers. The container content may be diluted in a volume of 5 to 5000 mL of diluent, preferably from 10 to 1000 mL of diluent, more preferably from 50 to 500 mL of diluent, even more preferably from 20 to 200 mL of diluent, most preferably around 100 mL of diluent.

Reconstituted embodiments of the present invention can further be diluted if so desired. This further dilution is preferably carried out with an aqueous diluent as described herein. The reconstituted solution will be diluted depending on the concentration in the reconstituted solution and the desired concentration in the diluted solution.

In some embodiments, said pharmaceutical composition comprises a DHP-I inhibitor (e.g., cilastatin) as a single active ingredient.

In other embodiments, said pharmaceutical composition comprises a DHP-I inhibitor (e.g., cilastatin) and another drug, preferably another drug other than a carbapenem antibiotic, such as other than imipenem. The list of drugs which may be combined with a DHP-I inhibitor (e.g., cilastatin) in a pharmaceutical composition is not particularly limited. Illustrative non-limiting examples of drug combinations as well as other embodiments of the combination treatment are provided below.

In some embodiments, the DHP-I inhibitor or the pharmaceutical composition comprising thereof is delivered through a drug delivery system. Further details and embodiments are described herein below.

### Drug delivery system

In a fourth aspect, the invention pertains to a drug delivery system comprising a DHP-I inhibitor or the pharmaceutical composition comprising thereof as described herein, for use according to the first or the second aspects. In preferred embodiments, the drug delivery system is according to the fifth or sixth aspects.

A drug delivery system (DDS) can be defined as a formulation or a device that enables a drug, or agent to selectively reach its site of action without reaching the nontarget cells, organs, or tissues. DDS enable improving drug accumulation at focal sites and reducing systemic toxicity at non-target sites.

### Pharmaceutical composition or drug delivery system suitable for trespassing the blood brain barrier

In a fifth aspect, the invention provides a pharmaceutical composition comprising a DHP-I inhibitor and a pharmaceutically acceptable excipient, or a drug delivery system comprising a DHP-I inhibitor or the pharmaceutical composition comprising thereof as described herein, wherein the pharmaceutical composition or drug delivery system is suitable for trespassing the blood brain barrier (BBB) and/or the blood retinal barrier (BRB).

In some embodiments, the pharmaceutical composition or the drug delivery system suitable for trespassing the BBB and/or BRB is for oral or parenteral administration, such as intravascular (e.g., intravenous or intraarterial), subcutaneous, intramuscular, intraperitoneal, intraventricular or intraepidural administration. In preferred embodiments, this composition or drug delivery system is for intravenous administration.

In some embodiments, these drug delivery systems are brain-targeting delivery systems (Liu P. and Jiang C., 2022). In preferred embodiments, these include one or more of the following, as well as combinations thereof:
(a) modification of the drug or agent or nanoparticles comprising any thereof with a brain-targeting ligand. This can be a biological molecule such as peptides, proteins or antibodies. An example would be the PepC7 and TGN peptides described by Guo Xu et al. 2020. Other biological ligands may be endogenous substances such as ApoE which naturally are transported through the BBB. Other peptides or proteins used for overcoming BBB are ApoA-I, angiopep-2, T7, and lactoferrin. Additional brain-targeting ligands are antibodies against human insulin receptor (HIR), designated as HIRMab, or against the TfR, designated as TfRMab. These may be used for instance for creating IgG fusion proteins (Pardridge WM, 2015).
(b) modification of the drug or agent or nanoparticles comprising any thereof with a precise targeting ligand, such as peptides targeting RAGE (e.g., in Alzheimer disease), the DAG peptide recognizing cerebrovascular changes and the Tet1 targeting neurons.
(c) modification of the drug or agent or nanoparticles comprising any thereof with substances inhibiting P-gp efflux, such as Tween-80.

The nanoparticles may be obtained using a large number of molecules well known in the art, including, but not limited to, organic or inorganic charged ions or a combination thereof. In some embodiments, the nanoparticle comprises an elemental metal, alloy comprising a metal, or a metal species-containing compound, the metal is preferably Cd, Zn, Cu, Pb, Ag, Mn, Ni, Au, Mg, Fe, Hg, Pt or a combination or alloy of one or more thereof. As used herein, by the term "metal species-containing compound" is meant a compound containing a metal or metalloid in any valence state. In various preferred embodiments, the nanoparticle comprises semiconductor crystals, including, but not limited, to CdS, CdSe, CdTe, ZnS, ZnSe, ZnTe, PbS, PbSe, PbTe, Cul, HgS, HgSe, and HgTe. These semiconductors can be ternary or quaternary semiconductors, including, but not limited to, CdTe/S, CdSe/S, CdTe/Se, Cd/ZnTe, Cd/ZnSe/Te, and the like. In various preferred embodiments, the nanoparticle comprises oxides, such as ZnO, SnO₂, CoO, NiO, CdO, InO₂, and the like. In various preferred embodiments, the nanoparticle comprises more complex systems, including alloys such as Ag/Au, Ag/Cu, Au/Cu, phosphates such as LiFePO₄, chromates such as PbCrO₄, and the like.

In some embodiments, the nanoparticle may be surrounded by at least one stabilizer moiety. A stabilizer moiety can be any molecule capable of collapse that contains units of monomers, that can be synthetic or naturally occurring and can be linear, branched, hyperbranched, and/or dendrimeric. In preferred embodiments, the stabilizing moiety comprises one or more polymers with ionizable or ionized groups. An ionizable moiety or group is any chemical functional group that can be rendered charged by adjusting solution conditions, while ionized moieties refers to chemical functional groups that are charged regardless of solution conditions. An ionizable moiety also includes any chemical functional group that can be rendered charged by the use of radiation or by the use of a static electromagnetic field. The ionized or ionizable moiety or group can be either cationic or anionic, and can be continuous along an entire chain as in the case of regular polymers, or can be interrupted by blocks containing different functional groups, as in the case of block polymers.

Examples of polymer stabilizers suitable in various embodiments include, but are not limited to, polyelectrolytes such as, e.g., poly (poly (acrylic acid), poly (styrene sulfonate), poly (diallyldimethylammonium chloride), poly (allylamine hydrochloride) (PAH), or others. Suitable examples of adsorbates include similar polyelectrolytes. In various preferred embodiments employing an adsorbate, the polymer stabilizer is of a larger molecular weight than the adsorbate moieties. In various embodiments, a preferred cationic group is the amino group and preferred anionic groups are carboxylic acid, sulfonic acid, phosphates, and the like. For cationic polymers, examples include, but are not limited to, poly (allylamine), poly (ethyleneimine), poly (poly (diallyldimethylammonium chloride), poly (arginine), chitosan, cationic collapsible proteins, poly (methacrylamido propyl trimethyl ammonium chloride) and poly (lysine). For anionic polymers, examples include, but are not limited to, poly (acrylic acid), poly (styrene sulfonic acid), poly (glutamic acid), poly (methacrylic acid), poly (aspartic acid), nucleic acids, anionic collapsible proteins, poly (anetholesulfonic acid), cellulose, poly (maleic acid) poly (vinyl phosphoric acid), etc. Block polymers are made up of blocks of polymers having different functional groups. The block polymers can be made up of blocks of any of the mentioned anionic and cationic polymers and another polymer that imparts a specific desirable property to the block polymer.

### Pharmaceutical composition or drug delivery system suitable for administration using a route of administration bypassing the blood brain barrier

In a sixth aspect, the invention relates to a pharmaceutical composition comprising a DHP-I inhibitor and a pharmaceutically acceptable excipient, or a drug delivery system comprising a DHP-I inhibitor or the pharmaceutical composition comprising thereof, wherein the pharmaceutical composition or drug delivery system is suitable for administration by a route bypassing the BBB, preferably selected from intrathecal, intracranial, intranasal and/or ocular administration, including topical, intraocular and/or periocular administration.

The different routes for ocular drug administration have been described herein above. Intraocular drug delivery includes for instance intracameral and intravitreal injections. Periocular administration includes subconjunctival, retrobulbar, peribulbar, subtenon, posterior juxtascleral, transcleral or suprachoroidal administration. A preferred route of administration is the intravitreal administration.

In some embodiments, the pharmaceutical composition or drug delivery system uses one or more of the nanoencapsulation or conjugation strategies discussed under the fifth aspect.

In some embodiments, the DHP-I inhibitor is delivered through an intranasal delivery system which may comprise the use of mucoadhesive agents, nanoparticles, lipid based systems, coadministration with vasoconstrictors, etc, for improved delivery.

In some embodiments, the delivery system is an ocular delivery system, such as contact lenses, ocular implants, or microneedles. In preferred embodiments, the delivery system is an intraocular delivery system. To reach the posterior segment of the eye, intraocular delivery systems have been designed, such as encapsulated cell technologies, biodegradable intravitreal devices, stimulus-responsive and nano-enabled devices, injectable intravitreal devices, nanoparticles and microparticles (e.g. liposomes, targeted nanosystems and dendrimers), multi-reservoir intravitreal systems, scleral plugs, microneedles coupled with sustained-release components as hybrid systems for intravitreal delivery, non-biodegradable intravitreal devices and capsule drug ring devices (Pillay *et al.,* 2016).

Other intraocular drug delivery devices that have been investigated are a discoid intravitreal device, microelectromechanical device, an osmotic minipump device, a hyaluronic acid plug device, a micromachined drug delivery device and a doughnut-shaped minitablet device (DSMT) (Choonara *et al.,* 2010).

In other embodiment, the delivery system is an implantable device, such as an implantable cathether-pump system. A typically small pump can be surgically implanted subcutaneously and connected to a thin, flexible catheter threaded under the skin. Stereotactic imaging guides the surgical placement of a catheter precisely into an area of the brain or to the spinal cord (Spinazzi et al. 2022).

It is contemplated that any embodiment discussed in this specification can be implemented with respect to any medical use, pharmaceutical composition, method of treatment, method of manufacturing a medicament and combination therapies of the invention, and vice versa. It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications are herein incorporated by reference to the same extent as if each individual publication or patent application was specifically and individually indicated to be incorporated by reference.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of excludes any element, step, or ingredient not specified in the claim except for, e.g., impurities ordinarily associated with the element or limitation.

The term "or combinations thereof' as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, 10, 11, 12, 13, 14 or 15%. Preferably the term "about" means exactly the indicated value (± 0%).

The following examples serve to illustrate the present invention and should not be construed as limiting the scope thereof.

### EXAMPLES

### Example 1. - Material and Methods

### Drugs

Crystalline cilastatin (CIL) was provided by Sun Pharmaceutical Industries Limited, (Madhya Pradesh, India) and was dissolved in normal 0.9% saline (vehicle).

### Animals

72 male albino Swiss mice were purchased at Charles River Laboratories (Les Oncins, France). Mice arrived at the animal facilities in the School of Medicine of the Complutense University of Madrid (ANUC ES28079000086) with a mean weight of 36.81±1.29g. Animals were housed in pairs, in polycarbonate cages (55x32x18cm), with cotton and cardboard tubes as environmental enrichment and were maintained under controlled environmental conditions (temperature, 21±1°C and humidity, 60±10%), in a 12h light-dark cycle (lights on at 7:00) with free access to water and pelleted food . At arrival, mice were allowed one week of habituation in which animals were not manipulated.

### Experimental animal model of glaucoma

As described in Cuenca *et al.* (2010), the laser treatment was performed to induce ocular hypertension (OHT). Left eyes of anesthetized mice [mixture of medetomidine (0.26 mg/kg; Medetor^{®}, Virbac España S.A., Barcelona, Spain) and ketamine (75 mg/kg; Anesketin^{®}, Dechra Veterinary Products SLU, Barcelona, Spain), i.p.] were treated with a diode laser beam applied without any lens, on the episcleral and limbal veins of the left eye were cauterized by performing a mean of 55-76 burns with a diode laser beam applied without any lens; the parameters used were: 50-100 µm of spot size, for 0.5s, and at a power of 0.3 W. After the surgery, tobramycin (Tobrex^{®}; Alcon, Barcelona, Spain) was administered on to the eyes to prevent eye drying and infection, and animals were rapidly recovered by the administration of hydrochlorure of atipamezole (0.013mg/g; Antidorm^{®}, Calier, Barcelona, Spain), an additional eye drop was applied to both eyes, and animals were kept warm until total recovery.

Intraocular Pressure (IOP) was measured as previously reported (Fernández-Albarral *et al.,* 2021) by using a rebound tonometer (Tono-Lab, Tiolat, OY, Helsinki, Finland) in anesthetized mice (2% isoflurane, ISOFLO ^{®}, Zoetis SL, Spain under an oxygen flow of 1.5 L/min). IOP was measured in both eyes of animals from all the experimental groups. Each IOP value was obtained from 3 independent measurements, each one resulting from the automatic mean of 6 consecutive measures. IOP was measured at the beginning of the experiment, prior to any intervention (basal), and after the OHT-induction surgery at five temporal times: 1, 2, 3, 5 and 7 days after the surgery. IOP measurements were always taken at the same day time (10:00 am) to avoid possible variations due to circadian rhythms.

### Experimental design

72 animals were randomly distributed into the different experimental groups, and no differences in the initial body weight were observed between groups: 36.6±1.38g (NAÏVE); 37.48±1.04g (NAÏVE+CIL); 36.85±1.25g (OHT) and 36.54±1.38g (OHT+CIL). 48 mice were submitted to the unilateral laser surgery, half of them were sacrificed 3 days after surgery and the other half 7 days after surgery. Every operated mouse had a left eye exposed to OHT (OHT) while the other normotensive contralateral one (CONTRA) was considered as an additional experimental group. From each experimental group, half of the animals were assigned to the CIL group, whereas the other half received vehicle (VH, saline) injections. A group of 24 NAÏVE mice, not exposed to the surgery, was included in the study and was administered with CIL or VH. The number of animals in the NAÏVE group was halved since both eyes could be used as independent samples for the NAÏVE group, but each eye of the animals exposed to the surgery was employed for two independent experimental groups: OHT (left eye) and CONTRA (right eye) while the two eyes could be used in the NAÏVE control group. CIL powder was stored at 4°C and dissolved in saline (0.9% NaCl) at a dose of 60 mg/ml every day the drug was administered. Mice were injected with the drug intraperitoneally (i.p.), at a dose of 300 mg/kg (according to Im *et al.,* 2017), starting 2 days before the surgery, on the surgery day immediately after the OHT laser-induction, and thereafter every day after until sacrifice (mainly from 12:00 to 15:00). Depending upon the experimental group, animals received 6 or 10 injections. Control animals received the same number of saline injections (5 mL/kg, i.p.) that their counterparts. The experimental groups were NAÏVE+VH day 3, NAÏVE+CIL day 3, OHT+VH day 3, OHT+ CIL day 3, CONTRA+VH day 3, CONTRA+ CIL day 3; NAÏVE+VH day 7, NAÏVE+ CIL day 7, OHT+VH day 7, OHT+ CIL day 7, CONTRA+VH day 7, CONTRA+CIL day 7.

### Immunohistochemistry

For the immunohistochemical analysis, optical cups with their retinas were frozen sectioned, in 16 µm thick sagittal serial series, from nasal to temporal (Leica Biosystems, cryostat CM-3050, Heidelberger, Germany). Tissue sections were collected onto gelatin-coated slides (two sections per slide). Analyses were performed taking in consideration the spatial organization of the retina all along the X axis in three regions: Nasal (N), Central (C) and Temporal (T); and all along the Y axis in three zones: Superior (S), Central (C) and Inferior (I).

A total of 5 animals per experimental group and per sacrifice day were selected for immunohistochemical analyses. Slides containing the tissue sections were dried at room temperature for 10 minutes. Samples were then washed three times with immunohistochemistry buffer (IB), containing 0.5% Animal-Free Blocker^{®} and Diluent, R.T.U. (Vector Laboratories, Inc., USA, Ref. SP-5035) and 0.3% Triton TM X-100 (Sigma-Aldrich, USA, Ref. T8787) in 0.1M PBS (Sigma-Aldrich, USA, Ref. P4417), pH 7.4. A solution of IB containing 0.5% of H2O2 was used to block the endogenous peroxidase for 15 min at room temperature. Subsequently, sections were incubated overnight, at 4°C, with the corresponding primary antibody diluted in IB with an additional 0.5% universal serum. The day after, slides were washed three times in IB and incubated for 2h at room temperature with the secondary antibody diluted in IB (see Table 1 below for details on the antibodies used). After incubations, sections were washed three times in IB and then incubated for 90 min at room temperature with ABC Peroxidase Staining Kit (Thermo scientific, Ref. 32020), 1:250 dilution. The reaction product was revealed by incubating the sections with 0.45 mg/mL of 3, 3'-diaminobenzidine tetrahydrochloride hydrate (Sigma-Aldrich, USA, Ref. D5637) and 0.03% H2O2 in PBS. Finally, sections were coverslipped with Aquatex^{®} (Merck KGaE, Germany, Ref. 1.08562.0050) mounting medium. Each immunohistochemical assay contained three slides (N, C and T) per experimental group, making a total of 18 slides plus a negative control slide with no primary antibody. The primary antibodies were Rabbit (polyclonal) anti Iba-1 (Ref. 019-19471, Wako Chemicals) for microglial cells, and Rabbit-Anti GFAP (polyclonal) (Ref. Z0334, Dako) for macroglial cells, with the secondary antibody Goat-Anti-rabbit IgG (H+L) Biotinylated (Ref. 31820, Thermo Scientific), used against both primary antibodies. We also marked RGCs with Mouse monoclonal anti-Brn-3a (Ref. MAB1585, Millipore), and its secondary antibody Goat-Anti-mouse IgG(H+L) Biotinylated (Ref, BA9200, Vector Laboratories). The specificity of the antibodies was verified by controls lacking the primary antibody, producing no background.

**Table 1. Antibodies and concentrations**

| Cell population | Primary antibody | Secondary antibody |
|---|---|---|
| RGCs | Mouse monoclonal anti-Brn-3a (Ref. MAB1585, Millipore) [1:600] | Goat-Anti-mouse IgG(H+L) Biotinylated (Ref. BA9200, Vector Laboratories) [1:200] |
| Macroglia: Müller cells and astrocytes | Rabbit-Anti GFAP (polyclonal) (Ref. Z0334, Dako) [1:800] | Goat-Anti-rabbit IgG (H+L) Biotinylated (Ref. 31820, Thermo Scientific) [1:200] |
| Microglia | Rabbit (polyclonal) anti Iba-1 (Ref. 019-19471, Wako Chemicals) [1:500] | |
| RGCs: retinal ganglion cells; GFAP: glial fibrillary acidic protein; Iba-1: ionized calcium-binding adapter molecule 1. For each antibody the commercial distributor is indicated, together with the specific antibody reference and the concentration employed in the present study. | | |

### Statistical analysis

Data from the initial body weight were analyzed by using a one-way Analysis of variance (ANOVA). A three-way ANOVA has been applied, whenever possible, considering the day of sacrifice (2 levels: 3 days and 7 days post-OHT induction), surgical intervention (3 levels: NAÏVE, OHT and CONTRA eyes) and the pharmacological treatment (2 levels: vehicle and CIL) as independent factors. Although no effect of the day of sacrifice was revealed in the three-way ANOVA analysis, we preferred to independently analyze the results obtained from the two cohorts of animals, those sacrificed 3 days and 7 days after the laser-induced OHT. Basal, 3d and 7d IOP measurements were analyzed independently: basal IOP data were analyzed by a one-way ANOVA, whereas 3d and 7d IOP results were analyzed with Repeated Measures (RM) ANOVA. Sphericity was studied, and in case of sphericity not being found, the Greenhouse-Geisser correction was employed. In order to study the statistical differences between groups, a two-way ANOVA was performed on each day. Immunohistochemistry results, from Brn3a, GFAP and Iba-1 immunostaining, were separated by 3 or 7 days after sacrifice and a two-way ANOVA (considering surgical intervention and pharmacological treatment as independent factors) was performed. Post-hoc comparisons were performed by using the Tukey test. P-values below 0.05 were considered significant. Normal distribution and homoscedasticity were verified by using Saphiro-Wilk and Levene tests, respectively. Data are presented as the mean value and the standard deviation (SD) and have been analyzed by using the SPSS software (SPPS 27, IBM, USA). Figures have been prepared by using the GraphPad Prism version 8.0.2 (GraphPad Software, Inc., San Diego, USA).

### Results

### IOP measurements

All animals presented normative basal IOP measurements. IOP measurements significantly increased in the OHT laser-induced eyes, while no changes in IOP values were observed in the CONTRA eyes both in animals sacrificed 3 or 7 days after the surgery, nor changes were observed due to CIL administration (Figure 1).

### Brn3a analysis

In the cohort of animals sacrificed 3 days after the OHT-induction, the analysis of the whole retina revealed that total number of brain-specific homeobox/POU domain protein (Brn3a+) cells was significantly reduced in OHT eyes compared to both NAÏVE and CONTRA eyes (Figure 2).

The further detailed regional analysis of the retina (Figure 2) revealed a generalized decrease in Brn3a+ cells in the OHT eyes, in all subregions but the N-S, with effects being more marked in the C and T subregions. CIL showed a trend to attenuate the Brn3a expression reduction, and in the temporal-superior area the reduction in Brn3a+ cells observed in the OHT+VH eyes was no longer observed when CIL was administered.

In the cohort of animals sacrificed 7 days after the surgery, a similar effect was observed in the analysis of Brn3a+ cells in the whole retina with OHT eyes showing a significant reduction in the total number of Brn3a+ cells, which was no longer observed after CIL administration (Figure 2).

In the regional detailed study per retinal subregions an overall decrease in Brn3a expression observed in OHT eyes was found leaving the C-S, C-I and T-S areas with no significant differences with the NAÏVE eyes. CIL reversed most of the previously mentioned alterations over RGCs (Figure 2).

### GFAP analysis

The analysis of Glial fibrillary acidic protein (GFAP) expression in the whole retina of animals sacrificed 3 days after the OHT induction revealed that OHT eyes showed higher levels of GFAP than the control NAÏVE and CONTRA eyes; while cilastatin seemed to induce a generalized decrease in GFAP expression that was more evident in the OHT eyes (Figure 3). In the analysis per retinal subregions a trend for an increase in GFAP expression in all regions was observed, but this effect only achieved statistical significance in the C-I retina and all T areas, where the reported increase in GFAP expression disappeared after cilastatin treatment. (Figure 3).

In the cohort of animals sacrificed 7 days after the OHT induction, the analysis of GFAP expression within the whole retina revealed that OHT eyes express GFAP at a higher rate than the NAÏVE control eyes, and that the CIL administration diminished the GFAP expression in all groups (Figure 3). The regional analysis of these retinas rendered an overall activation of OHT eyes, only being significant effects in the N-S, T-S and T-I areas. GFAP expression was then analyzed across the retinal layers, considering the inner (ONFUGCL, IPL, INL) and outer (OPL, ONL, PL) retina separately. In the inner retina analysis for the 3 days after-laser cohort of animals, OHT eyes showed a higher GFAP expression than control NAÏVE and CONTRA eyes, CIL inducing a general reduction of GFAP expression (Figure 4). In particular, the areas with a higher GFAP expression in OHT eyes were all N-C, C-S, C-I and the temporal ones. Moreover, in the C-S temporal areas the OHT+VH groups had a significantly higher GFAP expression compared to NAÏVE+VH, while the OHT+CIL eyes did not differ from NAÏVE+CIL. There was a general reduction in the GFAP expression in N-S and C-C due to the cilastatin treatment (Figure 4). In the outer retina GFAP was only augmented in some temporal subregions, in these subregions the drug, cilastatin, avoided such an increment in GFAP.

In the cohort of animals sacrificed 7 days after the OHT induction, the regional study of the inner retina OHT eyes showed elevations in GFAP levels in the N-S, N-I, C-C, T-S and T-I retina with no changes in the outer retina. CIL induced a general decrease in GFAP expression in most regions of the inner and outer retina (Figure 4).

### Iba-1 analysis

The inventors observed a remarkable increase in the amount of Iba-1 + cells in the total retina of OHT+VH animals both at 3 and 7 days after the surgery, observing a generalized response in the 9 retinal areas at day 3, but with a more maintained microglial response in the central and temporal regions of the retina at day 7. Milder but similar changes in the CONTRA eyes were appreciated in the total retinal analysis at both time points, and the further regional analysis revealed a higher microglial activation in the areas of the retina closer to the optic disc (nasal and central areas) 3 days after the OHT induction, with more moderate changes at day 7. CIL seemed to attenuate the effects of the OHT induction in both OHT and CONTRA eyes at both time points. In the further regional and layer analyses CIL seemed to revert most of these changes, suggesting anti-inflammatory effects, diminishing the microglial response in the retina. Even though having a generalized response over all the retina, the effects appear to be more remarkable in the areas closer to the optic disc, returning the microglial activation to values near to the ones observed in NAÏVE animals (Figure 5).

The amount of microglial cells in different retinal layers was analyzed at 3 and 7 days after the surgery. A higher amount of lba-1 + cells in the ONFL-CGL, followed by IPL and OPL compared to the INL and ONL-PL. The inventors appreciated an increase in the Iba-1+ number of cells in OHT+VH eyes in each layer, with the most accused changes in the ONFL-GCL, concluding that this may be the most affected layer of the retina, especially at day 3. Regarding the analysis per retinal layers the CONTRA eyes did only differ from the NAÏVE eyes in both plexiform layers, but the same pattern was observed as in the OHT eyes: higher number of Iba-1+ cells in the ONFL-GCL, IPL and OPL (Figure 6). As it occurred in the general and regional analyses, CIL reversed most of these changes in the OHT and CONTRA eyes (Figure 6).

### REFERENCES

Ahmed S, Amin MM, Sayed S. Ocular Drug Delivery: a Comprehensive Review. AAPS PharmSciTech. 2023 Feb 14;24(2):66.
Berge SM, Bighley LD, Monkhouse DC. Pharmaceutical salts. J Pharm Sci. 1977 Jan;66(1):1-19.
Budhram-Mahadeo V, Morris PJ, Lakin ND, Dawson SJ, Latchman DS. The different activities of the two activation domains of the Brn-3a transcription factor are dependent on the context of the binding site. J Biol Chem. 1996 Apr 12;271(15):9108-13.
Choonara YE, Pillay V, Danckwerts MP et al (2010) A review of implantable intravitreal drug delivery technologies for the treatment of posterior segment eye diseases. J Pharm Sci 99:2219-2239
Clissold SP, Todd PA, Campoli-Richards DM. Imipenem/cilastatin. A review of its antibacterial activity, pharmacokinetic properties and therapeutic efficacy. Drugs. 1987 Mar;33(3):183-241. Cordeiro MF, Levin LA. Clinical evidence for neuroprotection in glaucoma. Am J Ophthalmol. 2011 Nov;152(5):715-6.
Cuenca, N., Pinilla, I., Fernández-Sánchez, L., Salinas-Navarro, M., Alarcón-Martínez, L., Avilés-Trigueros, M., de la Villa, P., Miralles de Imperial, J., Villegas-Pérez, M. P., & Vidal-Sanz, M. (2010). Changes in the inner and outer retinal layers after acute increase of the intraocular pressure in adult albino Swiss mice. Experimental Eye Research, 91(2), 273-285. Cunningham AJ, Murray CA, O'Neill LA, Lynch MA, O'Connor JJ. Interleukin-1 beta (IL-1 beta) and tumour necrosis factor (TNF) inhibit long-term potentiation in the rat dentate gyrus in vitro. Neurosci Lett. 1996 Jan 12;203(1):17-20.
von Ertzen. U., Egensperger R., Ko¨sel S., Grasbon-Frodal E. M., Imai Y., Bise K., Kohsaka S., Mehraein P. and Graeber M. B. (1998) Microglia and the development of spongiform change in Creutzfeldt-Jakob disease. J Neuropathol. Exp. Neurol. 57, 246-256.
Del Valle J, Noriega AR, Sanz F, Revilla AP, Otero JR. Efficacy and safety of imipenem/cilastatin in the empirical treatment of septicemia. Scand J Infect Dis Suppl. 1987;52:20-5.
DiSabato, D. J., Quan, N. & Godbout, J. P. Neuroinflammation: the devil is in the details. J. Neurochem. 139 (Suppl. 2), 136-153 (2016).
Dubald M, Bourgeois S, Andrieu V, Fessi H. Ophthalmic Drug Delivery Systems for Antibiotherapy-A Review. Pharmaceutics. 2018 Jan 13;10(1):10.
European Glaucoma Society Terminology and Guidelines for Glaucoma, 5th Edition. British Journal of Ophthalmology 2021;105:1-169.
European Medicines Agency. (02 June 2023). Recarbrio: EPAR - Product Information. https://www.ema.europa.eu/en/documents/product-information/recarbrio-epar-product-information_en.pdf
Flaxman, S. R., Bourne, R. R. A., Resnikoff, S., Ackland, P., Braithwaite, T., Cicinelli, M. V., Das, A., Jonas, J. B., Keeffe, J., Kempen, J., Leasher, J., Limburg, H., Naidoo, K., Pesudovs, K., Silvester, A., Stevens, G. A., Tahhan, N., Wong, T., Taylor, H., ... Zheng, Y. (2017).
Global causes of blindness and distance vision impairment 1990-2020: a systematic review and meta-analysis. The Lancet Global Health, 5(12), e1221-e1234.
Fernández-Albarral, Jose A., de Hoz, R., Matamoros, J. A., Chen, L., López-Cuenca, I., Salobrar-Garcia, E., Sánchez-Puebla, L., Ramirez, J. M., Triviño, A., Salazar, J. J., & Ramirez, A. I. (2022). Retinal Changes in Astrocytes and Müller Glia in a Mouse Model of Laser-Induced Glaucoma: A Time-Course Study. Biomedicines, 10(5).
Fernández-Albarral, José Antonio, Salazar, J. J., Hoz, R. de, Marco, E. M., Martín-Sánchez, B., Flores-Salguero, E., Salobrar-Garcia, E., López-Cuenca, I., Barrios-Sabador, V., Avilés-Trigueros, M., Valiente-Soriano, F. J., Miralles de Imperial-Ollero, J. A., Vidal-Sanz, M., Triviño, A., Ramirez, J. M., López-Gallardo, M., & Ramirez, A. I. (2021). Retinal molecular changes are associated with neuroinflammation and loss of rgcs in an experimental model of glaucoma. International Journal of Molecular Sciences, 22(4), 1-29.
Fletcher, A. (2011). Nerve cell function and synaptic mechanisms. Anesthesia and Intensive Care Medicine, 12(6), 253-257.
Food and Drug Administration. (02 June 2023). Recarbrio Label. https://www.accessdata.fda.gov/drugsatfda_docs/label/2019/212819s0001bl.pdf
Food and Drug Administration. (02 June 2023). Primaxin Label. http://www.accessdata.fda.gov/drugsatfda_docs/label/2008/050587s065,050630s0281bl.pdf
Fricker M, Tolkovsky AM, Borutaite V, Coleman M, Brown GC.Physiol Rev. 2018 Apr 1;98(2):813-880.
Galindo-Romero C, Avilés-Trigueros M, Jiménez-López M, Valiente-Soriano FJ, Salinas-Navarro M, Nadal-Nicolás F, et al. Axotomy-induced retinal ganglion cell death in adult mice: quantitative and topographic time course analyses. Experimental Eye Research. 2011;92(5):377-387.
Gazzard G, Konstantakopoulou E, Garway-Heath D, et al. Selective laser trabeculoplasty versus eye drops for first-line treatment of ocular hypertension and glaucoma (LiGHT): a multicentre randomised controlled trial. Lancet 2019; 393(10180):1505-16.
Glass CK, Saijo K, Winner B, Marchetto MC, Gage FH. Mechanisms underlying inflammation in neurodegeneration. Cell. 2010 Mar 19;140(6):918-34.
Goel P, Chakrabarti S, Goel K, Bhutani K, Chopra T, Bali S. Neuronal cell death mechanisms in Alzheimer's disease: An insight. Front Mol Neurosci. 2022 Aug 25; 15:937133.
Gomes, F. V., Llorente, R., Del Bel, E. A., Viveros, M. P., López-Gallardo, M., & Guimarães, F. S. (2015). Decreased glial reactivity could be involved in the antipsychotic-like effect of cannabidiol. Schizophrenia Research, 164(1-3), 155-163.
Guo, Q., Xu, S., Yang, P., Wang, P., Lu, S., Sheng, D., Qian, K., Cao, K., Cao, J., Lu, W., & Zhang, Q. (2020). A dual-ligand fusion peptide improves the brain-neuron targeting of nanocarriers in Alzheimer's disease mice. Journal of Controlled Release, 320, 347-362.
Gupta PK, Asrani S, Freedman SF, El-Dairi M, Bhatti MT. Differentiating glaucomatous from non-glaucomatous optic nerve cupping by optical coherence tomography. Open Neurol J. 2011 Jan 26;5:1-7..
Hammond, T. R., Marsh, S. E. & Stevens, B. Immune signaling in neurodegeneration. Immunity 50, 955-974 (2019).
Higuchi T. and Stella V. Pro-drugs as Novel Delivery Systems, Vol. 14 of the A.C.S. Symposium Series; in Bioreversible Carriers in Drug Design, ed. Edward B. Roche, American Pharmaceutical Association and Pergamon Press, 1987
Humanes B, Lazaro A, Camano S et al. Cilastatin protects against cisplatin-induced nephrotoxicity without compromising its anticancer efficiency in rats. Kidney Int 2012; 82: 652-663.
Humanes B, Camaño S, Lara JM, Sabbisetti V, González-Nicolás MÁ, Bonventre JV, Tejedor A, Lázaro A. Cisplatin-induced renal inflammation is ameliorated by cilastatin nephroprotection. Nephrol Dial Transplant. 2017 Oct 1;32(10):1645-1655..
Im, D. sig, Shin, H. jin, Yang, K. J., Jung, S. Y., Song, H. yeon, Hwang, H. S., & Gil, H. W. (2017). Cilastatin attenuates vancomycin-induced nephrotoxicity via P-glycoprotein. Toxicology Letters, 277(December 2016), 9-17.
Ji RR, Xu ZZ, Gao YJ. Emerging targets in neuroinflammation-driven chronic pain. Nat Rev Drug Discov. 2014 Jul;13(7):533-48.
Jonas, J. B., Aung, T., Bourne, R. R., Bron, A. M., Ritch, R., & Panda-Jonas, S. (2017). Glaucoma. The Lancet, 390(10108), 2183-2193.
Kabbara WK, Meski MM, Ramadan WH, Maaliki DS, Salameh P. Adherence to International Guidelines for the Treatment of Uncomplicated Urinary Tract Infections in Lebanon. Can J Infect Dis Med Microbiol. 2018;2018 7404095.
Kang JM, Tanna AP. Glaucoma. Med Clin North Am. 2021 May;105(3):493-510.
Kaur G, Singh NK. Inflammation and retinal degenerative diseases. Neural Regen Res. 2023 Mar;18(3):513-518.
Kuo Bl, Fung CP, Liu CY. Meropenem versus imipenem/cilastatin in the treatment of sepsis in Chinese patients. Zhonghua Yi Xue Za Zhi (Taipei). 2000 May;63(5):361-7.
Kwon, H.S., Koh, SH. Neuroinflammation in neurodegenerative disorders: the roles of microglia and astrocytes. Transl Neurodegener 9, 42 (2020).
Lago, M.D. & Bengoa, A. (2018). Epidemiologia: el glaucoma del siglo XXI . Boletin de la Sociedad Oftalmológica de Madrid 58. https://sociedadoftalmologicademadrid.com/boletin-de-la-sociedad oftalmologica-de-madrid-n-o-58-2018/
Libraries Michigan State University (24 August 2023). Visual system: central processing. https://openbooks.lib.msu.edu/introneuroscience1/chapter/vision-central-processing/
Liu B, Hong JS. Role of microglia in inflammation-mediated neurodegenerative diseases: mechanisms and strategies for therapeutic intervention. J Pharmacol Exp Ther. 2003 Jan;304(1):1-7..
Liu P, Jiang C. Brain-targeting drug delivery systems. Wiley Interdiscip Rev Nanomed Nanobiotechnol. 2022 Sep;14(5):e1818.
Lyman M, Lloyd DG, Ji X, Vizcaychipi MP, Ma D. Neuroinflammation: the role and consequences. Neurosci Res. 2014 Feb;79:1-12.
Marchesi N, Fahmideh F, Boschi F, Pascale A, Barbieri A. Ocular Neurodegenerative Diseases: Interconnection between Retina and Cortical Areas. Cells. 2021 Sep 12;10(9):2394. Marinelli S, Maiarù M, Colciago A. Editorial: Neuroinflammation and Neuroautoimmunity in Peripheral Neuropathies: Old Players, New Roles. Front Immunol. 2021 Nov 23;12:801760. McCann P, Hogg R, Wright DM, et al. Glaucoma in the Northern Ireland Cohort for the Longitudinal Study of Ageing (NICOLA): cohort profile, prevalence, awareness and associations. Br J Ophthalmol 2020 Feb 7; bjophthalmol-2019-315330.
Mélik Parsadaniantz, S., Réaux-le Goazigo, A., Sapienza, A., Habas, C., & Baudouin, C. (2020). Glaucoma: A Degenerative Optic Neuropathy Related to Neuroinflammation? Cells, 9(3), 1-14.
Nadal-Nicolás FM, Jiménez-López M, Sobrado-Calvo P, Nieto-López L, Cánovas-Martínez I, Salinas-Navarro M, et al Brn3a as a marker of retinal ganglion cells: qualitative and quantitative time course studies in naive and optic nerve-injured retinas. Investigative Ophthalmology & Visual Science. 2009;50(8):3860-3868.
Nagano R, Shibata K, Naito T, et al. Therapeutic efficacy of BO-3482, a novel dithiocarbamate carbapenem, in mice infected with methicillin-resistant Staphylococcus aureus. Antimicrobial Agents and Chemotherapy. 1997 Oct;41(10):2278-2281.
Nettis MA, Pariante CM. Is there neuroinflammation in depression? Understanding the link between the brain and the peripheral immune system in depression. Int Rev Neurobiol. 2020; 152:23-40.
Pardridge WM. Targeted delivery of protein and gene medicines through the blood-brain barrier. Clin Pharmacol Ther. 2015 Apr;97(4):347-61.
Persidsky Y, Ramirez SH, Haorah J, Kanmogne GD. Blood-brain barrier: structural components and function under physiologic and pathologic conditions. J Neuroimmune Pharmacol. 2006 Sep; 1 (3):223-36.
Prum BE Jr, Rosenberg LF, Gedde SJ, et al. Primary open-angle glaucoma preferred practice pattern® guidelines. Ophthalmology 2016; 123(1):P41-111.
Rafiei F, Tabesh H, Farzad F. Sustained subconjunctival drug delivery systems: current trends and future perspectives. Int Ophthalmol. 2020 Sep;40(9):2385-2401.
Ramirez, A. I., de Hoz, R., Fernández-Albarral, J. A., Salobrar-Garcia, E., Rojas, B., Valiente-Soriano, F. J., Avilés-Trigueros, M., Villegas-Pérez, M. P., Vidal-Sanz, M., Triviño, A., Ramirez, J. M., & Salazar, J. J. (2020). Time course of bilateral microglial activation in a mouse model of laser-induced glaucoma. Scientific Reports, 10(1), 1-17.
Rautio J, Kumpulainen H, Heimbach T, Oliyai R, Oh D, Järvinen T, Savolainen J. Prodrugs: design and clinical applications. Nat Rev Drug Discov. 2008 Mar;7(3):255-70.
Shinozaki, Y., & Koizumi, S. (2021). Potential roles of astrocytes and Müller cells in the pathogenesis of glaucoma. Journal of Pharmacological Sciences, 145(3), 262-267.
Smith MD, Dawson SJ, Latchman DS. The Brn-3a transcription factor induces neuronal process outgrowth and the coordinate expression of genes encoding synaptic proteins. Mol Cell Biol. 1997 Jan;17(1):345-54.
Spinazzi EF, Argenziano MG, Upadhyayula PS, Banu MA, Neira JA, Higgins DMO, Wu PB, Pereira B, Mahajan A, Humala N, Al-Dalahmah O, Zhao W, Save AV, Gill BJA, Boyett DM, Marie T, Furnari JL, Sudhakar TD, Stopka SA, Regan MS, Catania V, Good L, Zacharoulis S, Behl M, Petridis P, Jambawalikar S, Mintz A, Lignelli A, Agar NYR, Sims PA, Welch MR, Lassman AB, Iwamoto FM, D'Amico RS, Grinband J, Canoll P, Bruce JN. Chronic convection-enhanced delivery of topotecan for patients with recurrent glioblastoma: a first-in-patient, single-centre, single-arm, phase 1b trial. Lancet Oncol. 2022 Nov;23(11):1409-1418.
Stephenson J, Nutma E, van der Valk P, Amor S. Inflammation in CNS neurodegenerative diseases. Immunology. 2018 Jun;154(2):204-219.
Triviño A, Ramirez Al, Salazar JJ, de Hoz R, Rojas B, Padilla E, Tejerina T, Ramirez JM. A cholesterol-enriched diet induces ultrastructural changes in retinal and macroglial rabbit cells. Exp Eye Res. 2006 Aug;83(2):357-66.
Vallée A. Neuroinflammation in Schizophrenia: The Key Role of the WNT/β-Catenin Pathway. Int J Mol Sci. 2022 Mar 4;23(5):2810.
Vitrikas, K., Hurst, N. (2022). Disorders of the Peripheral Nervous System. In: Paulman, P.M., Taylor, R.B., Paulman, A.A., Nasir, L.S. (eds) Family Medicine. Springer, Cham.
Wang et al., Front. Pharmacol., 08 June 2022 Sec. Drug Metabolism and Transport Volume 13 - 2022 | https://doi.org/10.3389/fphar.2022.938813
Weinreb, R. N., Aung, T., & Medeiros, F. A. (2014). The pathophysiology and treatment of glaucoma: A review. JAMA - Journal of the American Medical Association, 311(18), 1901-1911.
Wilson DM 3rd, Cookson MR, Van Den Bosch L, Zetterberg H, Holtzman DM, Dewachter I. Hallmarks of neurodegenerative diseases. Cell. 2023 Feb 16;186(4):693-714.
Wong, J. H. C., Ma, J. Y. W., Jobling, A. I., Brandli, A., Greferath, U., Fletcher, E. L., & Vessey, K. A. (2022). Exploring the pathogenesis of age-related macular degeneration: A review of the interplay between retinal pigment epithelium dysfunction and the innate immune system. In Frontiers in Neuroscience (Vol. 16). Frontiers Media S.A.
Zhang W, Xiao D, Mao Q, Xia H. Role of neuroinflammation in neurodegeneration development. Signal Transduct Target Ther. 2023 Jul 12;8(1):267.

### ITEMS OF THE INVENTION

1. DHP-I inhibitor for use as a neuroprotective medicament.
2. DHP-I inhibitor for use in a method for the prophylactic and/or therapeutic treatment of a neuroinflammatory disease in a mammalian subject, wherein a neuroinflammatory disease is a neurological or mental disease of the central nervous system (CNS) and/or the peripheral nervous system (PNS), which pathogenesis involves primary or secondary neuroinflammation and may evolve into neurodegeneration.
3. The DHP-I inhibitor for use according to item 2, wherein the neuroinflammatory disease is a neurological disease, preferably selected from the group consisting of Alzheimer's disease, Parkinson's disease, amyotrophic lateral sclerosis, multiple sclerosis, ocular neurodegenerative diseases, primary tauopathies, frontotemporal dementia (FTD), synucleinopathies (e.g., Lewy body dementia [LBD] and multisystem atrophy [MSA]), Huntington's disease (HD) and related polyglutamine (polyQ) diseases (including spinocerebellar ataxias [SCA]), prion disease (PrD), traumatic brain injury (TBI), chronic traumatic encephalopathy (CTE), stroke, migraine, spinal cord injury (SCI) and autoimmune encephalitides, such as systemic lupus erythematosus, Hashimoto encephalopathy, Sydenham chorea, Behçhet disease, Rasmussen encephalitis and limbic encephalitis epilepsy.
4. The DHP-I inhibitor for use according to item 3, wherein the neuroinflammatory disease is an ocular neurodegenerative disease, preferably selected from the group consisting of glaucoma, age-related macular degeneration, diabetic retinopathy, retinitis pigmentosa and neuromyelitis optica spectrum disorders (NMOSD).
5. The DHP-I inhibitor for use according to item 4, wherein the ocular neurodegenerative disease is glaucoma, such as, (i) normotensive glaucoma or (ii) high pressure glaucoma, including high pressure glaucoma resistant to intraocular pression (IOP)-lowering treatment.
6. The DHP-I inhibitor for use according to any one of items 2 to 5, wherein, one or more of the following effects is achieved by the treatment:
   a) a prevention and/or decrease of neurodegeneration, and/or
   b) a prevention and/or decrease of glia-mediated neuroinflammatory response, such as by reducing the inflammatory response of microglial and/or macroglial cells.
7. The DHP-I inhibitor for use according to any one of items 2 to 6, wherein said subject is a human subject.
8. The DHP-I inhibitor for use according to any one of items 2 to 7, wherein the DHP-I inhibitor is administered by a systemic route of administration, preferably by the parenteral route, such as by the intramuscular, intraperitoneal, subcutaneous or intravascular route, more preferably by the intravascular route.
9. The DHP-I inhibitor for use according to any one of items 2 to 7, wherein the DHP-I inhibitor is administered by the intrathecal or intracranial route or locally in the eye by topical, intraocular, comprising intravitreal or intracameral administration, and/or periocular administration, such as by subconjunctival, retrobulbar, peribulbar, subtenon, posterior juxtascleral, transcleral or suprachoroidal administration, preferably by intravitreal administration.
10. The DHP-I inhibitor for use according to any one of items 2 to 9, wherein DHP-I inhibitor is administered by the parenteral route at a dosage of 10 to 100 mg/kg of body weight, preferably of 15 to 60 mg/kg body weight, more preferably of 25 to 40 mg/kg of body weight for a human adult.
11. The DHP-I inhibitor for use according to any one of items 2 to 10, wherein the DHP-I inhibitor is administered in combination with another treatment.
12. The DHP-I inhibitor for use according to item 11, wherein said disease is glaucoma and the other treatment is for treating pupillary block or an intraocular pressure-lowering treatment.
14. The DHP-I inhibitor for use according to any one of items 11 or 12, wherein the DHP-I inhibitor and the other treatment are simultaneously administered, preferably forming part of the same pharmaceutical composition.
15. The DHP-I inhibitor for use according to any one of items 11 or 12, wherein the DHP-I inhibitor and the other treatment are separately administered.
16. The DHP-I inhibitor for use according to any one of items 2 to 10, wherein the method comprises administering the DHP-I inhibitor as single agent.
17. A pharmaceutical composition comprising a DHP-I inhibitor and a pharmaceutically acceptable excipient; or a drug delivery system comprising a DHP-I inhibitor or the pharmaceutical composition comprising thereof, for use according to anyone of items 1 to 16.
18. A pharmaceutical composition comprising a DHP-I inhibitor and a pharmaceutically acceptable excipient, or a drug delivery system comprising a DHP-I inhibitor or the pharmaceutical composition comprising thereof, wherein the pharmaceutical composition or drug delivery system is suitable for trespassing the blood brain barrier (BBB) and/or the blood retinal barrier (BRB).
19. A pharmaceutical composition comprising a DHP-I inhibitor and a pharmaceutically acceptable excipient, or a drug delivery system comprising a DHP-I inhibitor or the pharmaceutical composition comprising thereof, wherein the pharmaceutical composition or drug delivery system is suitable for administration by a route bypassing the BBB, preferably selected from intrathecal, intracranial, intranasal and/or ocular administration, including topical, intraocular and/or periocular administration.
20. The drug delivery system according to item 19, wherein the delivery system is an ocular delivery system, such as contact lenses, ocular implants, or microneedles; or wherein the delivery system is an implantable device, such as an implantable cathether-pump system.
21. The DHP-I inhibitor for use according to any of items 1 to 16, the pharmaceutical composition or drug delivery system for use of item 17, or the pharmaceutical composition or drug delivery system of any of items 18 or 19, wherein the DHP-I inhibitor is cilastatin or a pharmaceutically acceptable salt thereof, preferably cilastatin sodium.

## Claims

1. DHP-I inhibitor for use as a neuroprotective medicament.

2. DHP-I inhibitor for use in a method for the prophylactic and/or therapeutic treatment of a neuroinflammatory disease in a mammalian subject, preferably a human subject, wherein a neuroinflammatory disease is a neurological or mental disease of the central nervous system (CNS) and/or the peripheral nervous system (PNS), which pathogenesis involves primary or secondary neuroinflammation and may evolve into neurodegeneration.

3. The DHP-I inhibitor for use according to claim 2, wherein the neuroinflammatory disease is a neurological disease, preferably selected from the group consisting of Alzheimer's disease, Parkinson's disease, amyotrophic lateral sclerosis, multiple sclerosis, ocular neurodegenerative diseases, primary tauopathies, frontotemporal dementia (FTD), synucleinopathies (e.g., Lewy body dementia [LBD] and multisystem atrophy [MSA]), Huntington's disease (HD) and related polyglutamine (polyQ) diseases (including spinocerebellar ataxias [SCA]), prion disease (PrD), traumatic brain injury (TBI), chronic traumatic encephalopathy (CTE), stroke, migraine, spinal cord injury (SCI) and autoimmune encephalitides, such as systemic lupus erythematosus, Hashimoto encephalopathy, Sydenham chorea, Behçhet disease, Rasmussen encephalitis and limbic encephalitis epilepsy.

4. The DHP-I inhibitor for use according to claim 3, wherein the neuroinflammatory disease is an ocular neurodegenerative disease, preferably selected from the group consisting of glaucoma, age-related macular degeneration, diabetic retinopathy, retinitis pigmentosa and neuromyelitis optica spectrum disorders (NMOSD).

5. The DHP-I inhibitor for use according to claim 4, wherein the ocular neurodegenerative disease is glaucoma, such as, (i) normotensive glaucoma or (ii) high pressure glaucoma, including high pressure glaucoma resistant to intraocular pression (IOP)-lowering treatment.

6. The DHP-I inhibitor for use according to any one of claims 2 to 5, wherein the DHP-I inhibitor is administered by a systemic route of administration, preferably by the parenteral route, such as by the intramuscular, intraperitoneal, subcutaneous or intravascular route, more preferably by the intravascular route.

7. The DHP-I inhibitor for use according to any one of claims 2 to 6, wherein the DHP-I inhibitor is administered by the intrathecal or intracranial route or locally in the eye by topical, intraocular, comprising intravitreal or intracameral administration, and/or periocular administration, such as by subconjunctival, retrobulbar, peribulbar, subtenon, posterior juxtascleral, transcleral or suprachoroidal administration, preferably by intravitreal administration.

8. The DHP-I inhibitor for use according to any one of claims 2 to 7, wherein the DHP-I inhibitor is administered in combination with another treatment.

9. The DHP-I inhibitor for use according to claim 8, wherein the DHP-I inhibitor and the other treatment are simultaneously administered, preferably forming part of the same pharmaceutical composition or wherein the DHP-I inhibitor and the other treatment are separately administered.

10. The DHP-I inhibitor for use according to any one of claims 2 to 7, wherein the method comprises administering the DHP-I inhibitor as single agent.

11. A pharmaceutical composition comprising a DHP-I inhibitor and a pharmaceutically acceptable excipient; or a drug delivery system comprising a DHP-I inhibitor or the pharmaceutical composition comprising thereof, for use according to anyone of claims 1 to 10.

12. A pharmaceutical composition comprising a DHP-I inhibitor and a pharmaceutically acceptable excipient, or a drug delivery system comprising a DHP-I inhibitor or the pharmaceutical composition comprising thereof, wherein the pharmaceutical composition or drug delivery system is suitable for trespassing the blood brain barrier (BBB) and/or the blood retinal barrier (BRB).

13. A pharmaceutical composition comprising a DHP-I inhibitor and a pharmaceutically acceptable excipient, or a drug delivery system comprising a DHP-I inhibitor or the pharmaceutical composition comprising thereof, wherein the pharmaceutical composition or drug delivery system is suitable for administration by a route bypassing the BBB, preferably selected from intrathecal, intracranial, intranasal and/or ocular administration, including topical, intraocular and/or periocular administration.

14. The drug delivery system according to claim 13, wherein the delivery system is an ocular delivery system, such as contact lenses, ocular implants, or microneedles; or wherein the delivery system is an implantable device, such as an implantable cathether-pump system.

15. The DHP-I inhibitor for use according to any of claims 1 to 10, the pharmaceutical composition or drug delivery system for use of claim 11, or the pharmaceutical composition or drug delivery system of any of claims 12 or 13, wherein the DHP-I inhibitor is cilastatin or a pharmaceutically acceptable salt thereof, preferably cilastatin sodium.
